# EUROPÄISCHE PATENTSCHRIFT

(11) **EP 3 049 173 B1**
(45) Veröffentlichungstag und Bekanntmachung des Hinweises auf die Patenterteilung: **03.01.2018**
(21) Anmeldenummer: 14749722.6
(22) Anmeldetag: 05.08.2014
(51) Int. Cl.: B01D 3/26, B01F 3/04, C07C 7/11, B01D 3/14, B01D 3/16, B01D 3/22, B01D 3/28, B01D 3/32, C10K 1/18, B01D 53/14, B01D 53/18

(54) **VERFAHREN ZUM REINIGEN EINES SPALTGASSTROMS IN EINER ÖLWASCHKOLONNE**
METHOD FOR CLEANING A FLOW OF CRACKED GAS IN AN OIL WASHING COLUMN
PROCÉDÉ DE NETTOYAGE D'UN FLUX DE GAZ DE CRAQUAGE DANS UNE COLONNE DE NETTOYAGE D'HUILE

(30) Priorität: 25.09.2013 EP 13004648
(43) Veröffentlichungstag der Anmeldung: 03.08.2016
(73) Patentinhaber: Linde Aktiengesellschaft, 80331 München (DE)
(72) Erfinder: ALZNER, Gerhard, 81476 München (DE); MATTEN, Christian, 82049 Pullach (DE)
(74) Vertreter: Meilinger, Claudia Sabine
(86) Internationale Anmeldenummer: PCT/EP2014/002146
(87) Internationale Veröffentlichungsnummer: WO 2015/043697

(56) Entgegenhaltungen:
- WO-A1-2013/149721
- DE-A1-102006 045 498
- DE-A1-102007 056 964
- US-A- 5 080 837
- US-A- 5 223 183
- US-A- 5 407 605

## Beschreibung

Die Erfindung betrifft ein Verfahren gemäß dem Anspruch 1.
Eine bei einem derartigen Verfahren verwendete Ölwaschkolonne weist in der Regel, wie die in dem Dokument DE 10 2006 045498 in Fig. 2 offenbarte Wasserwaschkolonne 2, einen entlang einer Längsachse erstreckten Mantel auf, der einen Innenraum der Kolonne begrenzt, wobei der Innenraum zumindest in einen oberen Abschnitt 2o und einen entlang der Längsachse darunter angeordneten, separaten unteren Abschnitt 2u unterteilt ist, wobei im oberen Abschnitt 2o mehrere, erste Einbauten und im unteren Abschnitt 2u mehrere, zweite Einbauten angeordnet sind.
In einer Ölwaschkolonne wird ein Spaltgasstrom zunächst durch die zweiten Einbauten (Stoffaustauschböden) im unteren, sogenannten Ölabschnitt und sodann durch die ersten Einbauten (Stoffaustauschböden) im oberen, sogenannten Benzinabschnitt geführt, wobei die zweiten Stoffaustauschböden mit einem flüssigen, kohlenwasserstoffhaltigen Waschmittel (insbesondere mit einer Ölfraktion) beaufschlagt werden, um einen Stoffaustausch zwischen dem besagten Spaltgasstrom und jenem zweiten Waschmittel herzustellen, so dass im Ölabschnitt eine Ölfraktion aus dem Spaltgasstrom abgetrennt wird, und wobei die ersten Stoffaustauschböden mit einem flüssigen, kohlenwasserstoffhaltigen ersten Waschmittel beaufschlagt werden (insbesondere mit einer Benzinfraktion), um einen Stoffaustausch zwischen dem Spaltgasstrom und jenem ersten Waschmittel herzustellen, so dass im Benzinabschnitt eine Benzinfraktion aus dem Spaltgas abgetrennt und von dort insbesondere abgezogen wird, die leichter-siedend ist als jene Ölfraktion.

Der Spaltgasstrom wird über Kopf aus dem Benzinabschnitt abgezogen, wobei in den Kopf des Benzinabschnitts bzw. der Kolonne eine Benzinfraktion zurückgeführt wird und dort als das erste Waschmittel verwendet wird. Bevorzugt kondensiert die in den Kopf zurückgeführte Benzinfraktion (so genannter Rücklauf) aus dem Spaltgas in einer der Ölwaschkolonne nachfolgenden Wasserwaschkolonne aus, gelangt aus dem Sumpf der Wasserwaschkolonne in einen Benzin-Wasser-Trennbehälter und wird von dort insbesondere wasserfrei als Rücklauf in den Kopf des Benzinabschnitts bzw. der Ölwaschkolonne gegeben. Fremdbenzin wird unter Umständen beim Anfahren der Anlage benötigt.

Kolonnen der eingangs genannten Art werden z.B. bei der Herstellung von Olefinen (wie z.B. Ethylen oder Propylen) zum Kühlen und Reinigen eines jene Olefine aufweisenden Spaltgasstromes verwendet, der durch thermische Spaltung eines kohlenwasserstoffhaltigen Einsatzes erzeugt wird. Die längerkettigen Kohlenwasserstoffe des kohlenwasserstoffhaltigen Einsatzes werden dabei unter Anwesenheit von Dampf thermisch in kürzerkettige Kohlenwasserstoffe (z.B. in die gewünschten Produkte Ethylen und/oder Propylen) aufgespalten. Derartige Verfahren werden als Steamcracken oder Pyrolyse von Kohlenwasserstoffen bezeichnet.

Die kohlenwasserstoffhaltigen Einsätze können dabei sehr verschieden hinsichtlich der Zusammensetzung und des Gemisches aus einzelnen längerkettigen Kohlenwasserstoffen sowie im Aggregatzustand sein. Es werden dabei sowohl gasförmige Einsätze als auch flüssige Einsätze gespalten, wobei die flüssigen Einsätze in der Regel höhere Anteile an längerkettigen Kohlenwasserstoffen und einen daraus resultierenden höheren Siedepunkt aufweisen. Als derartige flüssige Einsätze kommen beispielsweise Naphtha oder Gaskondensate in Frage. Ein typischer Naphthaeinsatz hat einen Siedepunkt im Bereich zwischen 160°C und 170 °C, während für gewöhnlich Gaskondensate einen Siedepunkt über 250 °C aufweisen.

Der kohlenwasserstoffhaltige Einsatzstoff (wie z.B. Naphtha) wird zur Dampfspaltung in die Konvektionszone eines Spaltofens geführt, insbesondere auf 550 °C bis 650 °C vorgewärmt und in die gasförmige Phase überführt. In der Konvektionszone wird dem kohlenwasserstoffhaltigen Einsatzdampf nunmehr heißer Prozessdampf zugegeben. Das gasförmige Gemisch aus kohlenwasserstoffhaltigem Einsatz und Wasserdampf wird aus der Konvektionszone in die beheizten Spalt- oder Pyrolyserohre des Spalt- oder Pyrolyseofens geführt. Im Inneren der beheizten Spaltrohre herrschen dabei insbesondere Temperaturen im Bereich von 800 °C bis 850 °C, die zur Aufspaltung der längerkettigen Kohlenwasserstoffe des Einsatzes in kürzerkettige, bevorzugt gesättigte Kohlenwasserstoffe, führen. Der zugegebene Prozessdampf dient dabei der Partialdruckerniedrigung der einzelnen Reaktionsteilnehmer, sowie der Verhinderung einer erneuten Aneinanderlagerung bereits gespaltener, kürzerkettiger Kohlenwasserstoffe. Die Verweilzeit in den Spaltrohren des Spaltofens beträgt dabei typischerweise zwischen 0,2 und 0,6 Sekunden.

Der mit einer Temperatur von ca. 850 °C aus dem Spaltofen austretende Spaltgasstrom besteht zum größten Teil aus Ethan, anderen Olefinen (Propen) und Diolefinen und wird rasch auf ca. 400 °C abgekühlt, um Sekundärreaktionen der sehr reaktionsfreudigen Spaltprodukte zu vermeiden. Der derart abgekühlte Spaltgasstrom wird als erstes der oben erläuterten Ölwaschkolonne zugeführt. Diese dient dazu, den erzeugten Spaltgasstrom weiter abzukühlen und in einem ersten Zerlegungsschritt eine Fraktion aus schwersiedenden Kohlenwasserstoffen (z.B. Ölfraktion bzw. Leichtölfraktion und Schwerölfraktion) sowie leichter-siedenden Kohlenwasserstoffen (z.B. Benzinfraktion) auszukondensieren und vom Spaltgas abzutrennen. Um insbesondere die Wärme des Spaltgases in der Anlage weiter zu nutzen, weist die abgetrennte Ölfraktion bzw. Schwerölfraktion eine Mindesttemperatur auf und wird in weiteren Prozessschritten der Anlage als Wärmeträger eingesetzt.

Das größte Problem derartiger Ölwaschkolonnen ist die Verschmutzung der einzelnen Stoffaustauschböden durch Polymerbildung (so genanntes Fouling). Dabei liegen der Polymerbildung im Wesentlichen zwei Mechanismen zugrunde.

Zum einen enthalten die kondensierenden Komponenten Monomere (dies sind beispielsweise ungesättigte Kohlenwasserstoffe wie Naphtene, Indene oder Styrene). Diese Monomere können unter bestimmten Bedingungen Polymere bilden. Diese Bedingungen wären polymerisationsfähiger Temperaturbereich, Auftreten der Monomere in hinreichend großer Konzentration, große Verweilzeit auf den Einbauten und die Anwesenheit von Rost. Diese Einflüsse werden als "fouling factors" bezeichnet. Optimalerweise ist das Zustandekommen aller vier Bedingungen zu verhindern.

Zum anderen verdampft der größte Teil der flüssigen Kohlenwasserstoffe, die im Benzinabschnitt als erstes Waschmittel aufgegeben werden, über den Benzinabschnitt auf dem Weg nach unten. Dies führt dazu, dass am unteren Ende des Benzinabschnitts die geringste Flüssigkeitsmenge und somit die größte Verweildauer der Flüssigkeit auf den Stoffaustauschböden bzw. -elementen vorhanden ist.

Zusätzlich steigen bei steigendem Rückfluss die Verdampfung der längerkettigen Kohlenwasserstoffe und damit die Temperatur des gasförmigen Kopfproduktes an.

Daher kommt es häufig bei Ölwaschkolonnen nach dem Stand der Technik zu Polymerbildung und zur Verlegung der unteren Stoffaustauschböden des Benzinabschnittes. Im Stand der Technik versucht man, diese Verschmutzungsproblematik zu verhindern, indem im Ölabschnitt side-to-side baffles verwendet werden und im Benzinabschnitt eine vergleichsweise große Anzahl an austauschwirksamen Stoffaustauschböden, z.B. in Form von Sieb- oder Ventilböden, so dass insgesamt eine Ölwaschkolonne von unvorteilhaft großer Bauhöhe bezüglich der Längsachse resultiert. Bei den side-to-side baffles bzw. Kolonnenböden handelt es sich um ein- oder mehrflutige, insbesondere geneigte (oder auch waagerechte) Kolonnenböden, die entlang der Längsachse der Ölwaschkolonne übereinander angeordnet sind und sich jeweils über einen Teil des Kolonnenquerschnitts erstrecken, wobei je zwei benachbarte, übereinander angeordnete side-to-side baffles versetzt zueinander angeordnet sind, so dass eine vom oberen Kolonnenboden abfließende flüssige Phase den darunter liegenden Kolonnenboden beaufschlägt. Die flüssige Phase fließt entsprechend auf ihrem Weg nach unten in der Ölwaschkolonne hin und her.

Der vorliegenden Erfindung liegt hiervon ausgehend die Aufgabe zugrunde, ein Verfahren zum Reinigen eines Spaltgasstroms in einer Ölwaschkolonne anzugeben, das dem vorgenannten Verschmutzungsrisiko entgegen wirkt.

Dieses Problem wird durch ein Verfahren mit den Merkmalen des Anspruchs 1 gelöst. Danach ist vorgesehen, dass das molare Verhältnis der in den Kopf des Benzinabschnitts pro Zeiteinheit zurückgeführten Stoffmenge der Benzinfraktion (in beispielsweise kmol/h), die dort als besagtes erstes Waschmittel verwendet wird, zu der pro Zeiteinheit in den Ölabschnitt eingeleiteten Stoffmenge des Spaltgases (in beispielsweise kmol/h) in einem Bereich von 1:16 bis 1:10, vorzugsweise 1:12 bis 1:10 liegt.

Wie bereits eingangs erläutert, besteht im unteren Teil des Benzinabschnittes, insbesondere auf den untersten Stoffaustauschböden des Benzinabschnitts der Ölwaschkolonne, die größte Gefahr einer Verschmutzung bzw. Verlegung durch Polymerbildung. Im Stand der Technik wurde insbesondere versucht, diesem Umstand durch eine Steigerung der Anzahl der Stoffaustauschböden (insbesondere in Form von Sieb- oder Ventilböden) im Benzinabschnitt entgegenzuwirken (siehe oben), was zu enormen Kolonnenlängen entlang der Längsachse führt. Versuche bzw. Untersuchungen zeigen jedoch, dass hierdurch der besagten Polymerbildung im Benzinabschnitt nicht wirksam entgegengetreten werden kann.

Der vorliegenden erfindungsgemäßen Lösung hingegen liegt die Erkenntnis zugrunde, dass ein mögliches Fouling nicht in dem von den Stoffaustauschböden her sensibleren Benzinabschnitt stattfinden darf, sondern im Ölabschnitt, dessen Stoffaustauschböden eine geringere Verschmutzungsanfälligkeit aufweisen.

Es hat sich diesbezüglich gezeigt, dass es günstig ist, das oben genannte erfindungsgemäße molare Verhältnis (kmol/h) von Benzin-Rücklauf am Kopf der Ölwaschkolonne zu Spaltgaseintritt im Sumpf der Ölwasschkolonne einzustellen, um ein Trockenlaufen der unteren ersten Stoffaustauschböden bzw. ein Auskondensieren der ungesättigten Kohlenwasserstoffe (insbesondere Monomere, wie z.B. Indene, Naphtene u.ä.) im Benzinabschnitt zu verhindern.

Grundsätzlich ist es vorteilhaft, die zurückgeführte Menge der Benzinfraktion so klein wie nötig zu halten. Eine zu große Menge erhöht die Temperatur der Kopfprodukte der Ölwaschkolonne. Auf dem Weg abwärts über die ersten Stoffaustauschböden verdampfen die längerkettigen Kohlenwasserstoffe. Das Spaltgasgemisch, nun angereichert um diese verdampften längerkettigen Kohlenwasserstoffe, hat dadurch einen höheren Siedepunkt als ohne diese verdampften Kohlenwasserstoffe, so dass die Gleichgewichtstemperatur auf dem obersten ersten Stoffaustauschboden ansteigt.

Zusammenfassend liegt also der vorliegenden Erfindung die Erkenntnis zugrunde, dass die Konzentration der ungesättigten Kohlenwasserstoffe (Monomere wie z.B. Indene, Napthene u.ä.) nicht im Benzinabschnitt stattfinden darf und die Zieltemperatur des Kopfproduktgases am ehesten erreicht wird, je weniger Benzinrücklauf am Kolonnenkopf aufgegeben werden kann.

Weiterhin werden vorliegend die oben beschriebenen austauschwirksamen zweiten Stoffaustauschböden eingesetzt, die zusätzlich die polymerisierfähigen, ungesättigten Kohlenwasserstoffe von den ersten Stoffaustauschböden (auch als Benzinböden bezeichnet) fernhalten.

Eine Vergrößerung der Anzahl der Stoffaustauschböden im Benzinteil mindert das Polymerisationsproblem hingegen nicht.

Bei den eingangs genannten Siebböden handelt es sich um Kolonnenböden mit einem Ablaufschacht, über den das Waschmittel auf darunter befindliche Kolonnenböden gelangt, wobei Siebböden eine Mehrzahl an Durchgangsöffnungen aufweisen, durch die das Spaltgas strömt und das auf dem jeweiligen Siebboden befindliche Waschmittel kontaktiert. Glockenböden können ebenfalls einen Ablaufschacht aufweisen. Weiterhin sind bei Glockenböden die Durchgangsöffnungen von Kaminhälsen eingefasst, die von Hauben überdacht sind, wobei insbesondere die Kaminhälse in die jeweils zugeordnete Haube hinein ragen. Ventilböden können ebenfalls einen Ablaufschacht aufweisen. Des Weiteren können bei Ventilböden die besagten Durchgangsöffnungen durch Ventile, insbesondere bewegliche wie auch feststehende Klappen oder Deckel, verschlossen werden, die bei hinreichendem Spaltgasdruck aufgedrückt werden, so dass das Spaltgas von unten nach oben durch die Durchgangsöffnungen strömen kann.

Weiterhin erstreckt sich vorzugsweise der den Sumpf der Ölwaschkolonne aufweisende Ölabschnitt entlang der Längsachse hin zum Benzinabschnitt, der sich an den Ölabschnitt anschließt (z.B. über den besagten Kaminboden), wobei der Kopf des Benzinabschnitts den Kopf der Ölwaschkolonne bildet, aus dem der abgekühlte und gereinigte Spaltgasstrom abziehbar ist.

Die besagte Längsachse der Ölwaschkolonne bzw. des Mantels der Ölwaschkolonne erstreckt sich bevorzugt - bezogen auf eine bestimmungsgemäß angeordnete und betriebsbereite Ölwaschkolonne - entlang der Vertikalen. Der Mantel der Ölwaschkolonne ist zumindest abschnittsweise zylindrisch ausgebildet, wobei die Längsachse der Ölwaschkolonne in diesem Fall mit der Zylinderachse des Mantels zusammenfällt.

Gemäß einer Ausführungsform der Erfindung kann vorgesehen sein, dass ein Teil der aus dem Benzinabschnitt abgezogenen Benzinfraktion auf einen der ersten Stoffaustauschböden zurückgeführt wird (so genannter pump-around), und zwar - bezogen auf eine bestimmungsgemäß angeordnete Ölwaschkolonne - vorzugsweise direkt auf einen der unteren ersten Stoffaustauschböden.

Bevorzugt handelt es sich bei jenem unteren ersten Stoffaustauschboden um den zweituntersten, drittuntersten oder viertuntersten ersten Stoffaustauschboden.

Weiterhin wird bevorzugt die Benzinfraktion von einem Kaminboden abgezogen, der den Benzinabschnitt vom Ölabschnitt abtrennt, oder von einem untersten ersten Stoffaustauschboden, der den Benzinabschnitt vom Ölabschnitt abtrennt.

Bevorzugt weist die Ölwaschkolonne an einem unteren Bereich des Ölabschnitts einen Einlass zum Einlassen des Spaltgasstromes auf, wobei die Ölwaschkolonne - sofern ein nicht weiter unterteilter Ölabschnitt bzw. eine 1-Kreis-Ölwäsche vorhanden ist - vorzugsweise so ausgebildet ist, dass der eingespeiste Spaltgasstrom in der Ölwaschkolonne durch die im Ölabschnitt übereinander angeordneten zweiten Stoffaustauschböden aufsteigt.

Gemäß einer weiteren Ausführungsform der Erfindung ist vorgesehen, dass der Ölabschnitt in einen Leichtölabschnitt und einen entlang der Längsachse darunter angeordneten, separaten Schwerölabschnitt unterteilt ist, wobei insbesondere der Leichtöl- und der Schwerölabschnitt mittels eines Kaminbodens voneinander getrennt sind. Ein Schwerölabschnitt wird insbesondere zur Behandlung von Spaltgasströmen eingesetzt, der durch Dampfspaltung von schwereren flüssigen Einsätzen (z.B. schwerer als Naphtha) gewonnen wurden. Mit anderen Worten kann der zweite Abschnitt also als einheitlicher Ölabschnitt ausgebildet sein (siehe oben) oder in einen Leichtöl- und einen Schwerölabschnitt unterteilt sein, in denen anstelle einer Ölfraktion eine Leichtöl- bzw. eine Schwerölfraktion anfallen.

Vorzugsweise wird bei Vorhandensein eines Schwerölabschnittes der Spaltgasstrom über den nunmehr im unteren Bereich des Schwerölabschnitts vorgesehenen Einlass der Ölwaschkolonne in die Kolonne eingeleitet und durch die zweiten Stoffaustauschböden im Schwerölabschnitt und sodann durch die zweiten Stoffaustauschböden im Leichtölabschnitt geführt, wobei die zweiten Stoffaustauschböden im Schwerölabschnitt mit einem flüssigen, kohlenwasserstoffhaltigen dritten Waschmittel beaufschlagt werden, um einen Stoffaustausch zwischen dem durch die zweiten Stoffaustauschböden geführten Spaltgastrom und jenem dritten Waschmittel herzustellen, wobei bevorzugt schwerere Kohlenwasserstoffe aus dem Spaltgasstrom abgetrennt werden, die sich im Sumpf der Ölwaschkolonne als Schwerölfraktion sammeln.

Weiterhin werden bevorzugt die zweiten Stoffaustauschböden im Leichtölabschnitt mit einem flüssigen, kohlenwasserstoffhaltigen zweiten Waschmittel beaufschlagt, um einen Stoffaustausch zwischen dem durch die zweiten Stoffaustauschböden geführten Spaltgasstrom und jenem zweiten Waschmittel herzustellen. Hierbei werden im Vergleich zum Benzinabschnitt (siehe oben) bevorzugt vorwiegend schwerer-siedende Kohlenwasserstoffe bzw. im Vergleich zum Schwerölabschnitt vorwiegend leichter-siedende Kohlenwasserstoffe aus dem Spaltgasstrom abgetrennt, die sich im Leichtölabschnitt als Leichtölfraktion sammeln. Diesbezüglich wird vorzugsweise jene Leichtölfraktion aus dem Leichtölabschnitt abgezogen (z.B. von einem Kaminboden, der Leichtöl- und Schwerölabschnitt voneinander trennt), abgekühlt und ggf. von Kokspartikeln gereinigt sowie zumindest teilweise in den Leichtölabschnitt als zweites Waschmittel bzw. Bestandteil des zweiten Waschmittels zurückgeführt.

Weiterhin wird bevorzugt ein Teil der aus dem Benzinabschnitt abgezogenen Benzinfraktion der aus dem Leichtölabschnitt abgezogenen Leichtölfraktion hinzugegeben und dieses Gemisch als zweites Waschmittel in den Leichtölabschnitt zurückgeführt und dort auf einen obersten zweiten Stoffaustauschboden erneut aufgegeben (analog zum Ölabschnitt ohne Schwerölabschnitt).

Weiterhin wird bevorzugt die im Sumpf der Ölwaschkolonne anfallende Schwerölfraktion abgezogen und nach Abkühlung und ggf. nach Entfernung von Kokspartikeln zumindest teilweise als drittes Waschmittel in den Schwerölabschnitt zurückgeführt und erneut auf einen obersten zweiten Stoffaustauschboden des Schwerölabschnitts aufgegeben.

Vorzugsweise beträgt die Länge des Benzinabschnitts nicht mehr als die Hälfte des Ölabschnitts, der ggf. einen Leichtöl- sowie einen Schwerölabschnitt aufweisen kann (siehe oben).

Weiterhin ist gemäß einer Ausführungsform der Erfindung vorgesehen, dass der Benzinabschnitt 6 bis 8, insbesondere 7, erste Stoffaustauschböden aufweist. Vorzugsweise weisen dabei benachbarte erste Stoffaustauschböden einen Abstand zueinander entlang der Längsachse im Bereich von 500mm bis 900mm auf.

Gemäß einer weiteren Ausführungsform ist vorgesehen, dass der Ölabschnitt der Ölwaschkolonne 10 bis 20, insbesondere 16, zweite Stoffaustauschböden aufweist. Für den Fall, dass der besagte Ölabschnitt in den besagten Leichtölabschnitt und jenen Schwerölabschnitt unterteilt ist, ist bevorzugt vorgesehen, dass der Leichtölabschnitt 6 bis 12, insbesondere 8, zweite Stoffaustauschböden aufweist, wohingegen der Schwerölabschnitt vorzugsweise 4 bis 8, insbesondere 8, zweite Stoffaustauschböden aufweist.

Vorzugsweise weisen benachbarte zweite Stoffaustauschböden des zweiten Ölabschnittes bzw. des Leicht- und Schwerölabschnittes der Ölwaschkolonne einen Abstand entlang der Längsachse im Bereich von 600mm bis 900mm auf.

Zum Verteilen des jeweiligen Waschmittels auf die zugeordneten zweiten Stoffaustauschböden weist die Ölwaschkolonne gemäß einer Ausführungsform der Erfindung einen im Ölabschnitt angeordneten ersten Flüssigkeitsverteiler auf, mit dessen Hilfe die zweiten Stoffaustauschböden mit jenem zweiten Waschmittel beaufschlagt werden.

Gemäß einem Ausführungsbeispiel der Erfindung wird mittels des ersten Flüssigkeitsverteilers jenes zweite Waschmittel auf die Kanten der Ablaufelemente eines obersten zweiten Stoffaustauschbodens des Ölabschnitts aufgegeben, so dass jenes zweite Waschmittel zu beiden Seiten der jeweiligen Kante über die Ablaufflächen vom jeweiligen Ablaufelement abfließt.

Für den Fall, dass der Ölabschnitt einen Leichtölabschnitt und einen Schwerölabschnitt aufweist, ist vorgesehen, dass der besagte erste Flüssigkeitsverteiler im Leichtölabschnitt angeordnet ist und jener oberste zweite Stoffaustauschboden des Ölabschnitts ein oberster zweiter Stoffaustauschboden des Leichtölabschnitts ist.

Bevorzugt ist ein zweiter Flüssigkeitsverteiler im Schwerölabschnitt angeordnet, mit dessen Hilfe die zweiten Stoffaustauschböden des Schwerölabschnitts mit dem dritten Waschmittel beaufschlagt werden. Mittels des zweiten Flüssigkeitsverteilers wird dabei bevorzugt jenes dritte Waschmittel auf die Kanten der Ablaufelemente eines obersten zweiten Stoffaustauschbodens des Schwerölabschnitts aufgegeben, so dass jenes dritte Waschmittel zu beiden Seiten der jeweiligen Kante über die Ablaufflächen vom jeweiligen Ablaufelement abfließt.

Vorzugsweise weisen der erste und/oder der zweite Flüssigkeitsverteiler jeweils eine Mehrzahl an Auslässen auf, durch die hindurch das jeweilige Waschmittel auf die Kanten der Ablaufelemente des jeweiligen obersten zweiten Stoffaustauschbodens gegeben wird, wobei die Auslässe jeweils lotrecht oberhalb einer Kante eines zugeordneten Ablaufelementes angeordnet sind.

Die zweiten Stoffaustauschböden des Ölabschnitts bzw. des Leichtöl- und Schwerölabschnitts sind vorzugsweise so übereinander angeordnet, dass die Ablaufelemente zweier benachbarter zweiter Stoffaustauschböden versetzt zueinander angeordnet sind, so dass das jeweilige Waschmittel, das von einer Ablauffläche eines oberen Ablaufelementes abfließt, auf eine Ablauffläche eines darunter versetzt angeordneten unteren Ablaufelementes auftrifft. Hierdurch ist mit Vorteil gewährleistet, dass die volle Flüssigkeitsmenge bereits am obersten Stoffaustauschboden zur Verfügung steht, im Gegensatz zu Sprühdüsen, bei denen regelmäßig 30% bis 50% der Flüssigkeit in den Spalten bzw. Durchgangsöffnungen zwischen den Ablaufelementen landet und erst auf tieferen Stoffaustauschböden wirksam wird.

Vorzugsweise wird mittels des ersten bzw. des zweiten Flüssigkeitsverteilers die flüssige Phase bzw. das jeweilige Waschmittel vollständig auf die Ablaufelemente, insbesondere deren Kanten, aufgegeben. Weiterhin ist der Flüssigkeitsaustrag über den Kopf der jeweiligen Kolonne bei versprühtem Waschmittel (kleine Tropfen) unvorteilhafter Weise größer.

Gemäß einer Ausführungsform der Erfindung weist der erste und/oder der zweite Flüssigkeitsverteiler eine Mehrzahl an längs erstreckten Endverteilerkanälen auf, die sich jeweils quer zur Längsachse bzw. entlang des Kolonnenquerschnitts sowie quer zu den Ablaufelementen bzw. deren jeweiliger Längserstreckungsrichtung über den im Wesentlichen gesamten Kolonnenquerschnitt erstrecken.

Vorzugsweise weisen die besagten Endverteilerkanäle jeweils einen quer zur Längsachse bzw. entlang des Kolonnenquerschnitts erstreckten Boden und zwei davon abgehende längserstreckte, einander gegenüber liegende Seitenwände auf, wobei die besagten Seitenwände jeweils einen oberen Rand aufweisen, und wobei die Auslässe in Form von insbesondere rechteckförmigen Ausnehmungen an den beiden oberen Rändern ausgebildet sind. Die Endverteilerkanäle sind des Weiteren stirnseitig bevorzugt je durch eine weitere Seitenwand begrenzt. Die besagten Ausnehmungen oder Ausschnitte an den Rändern der Seitenwände weisen dabei insbesondere jeweils eine Unterkante auf, über die die flüssige Phase bzw. das jeweilige Waschmittel aus dem jeweiligen Endverteilerkanal auf die darunter liegende Kante eines Ablaufelementes geleitet wird, wobei jene Unterkante entlang der Längsachse der Ölwaschkolonne zum jeweiligen Boden des betrachteten Endverteilerkanals beabstandet ist, so dass der jeweilige Endverteilerkanal theoretisch bis hin zu den besagten Unterkanten der Auslässe Verschmutzungen ansammeln kann, wobei mittels des jeweiligen Endverteilerkanals dann immer noch die zu verteilende flüssige Phase definiert über die Auslässe bzw. Ausnehmungen auf den zugeordneten zweiten Stoffaustauschboden gegeben werden kann.

Weiterhin weist der erste und/oder der zweite Flüssigkeitsverteiler zumindest zwei zueinander parallele, entlang der Längsachse des Kolonnenmantels oberhalb der Endverteilerkanäle angeordnete, längs erstreckte Vorverteilerkanäle auf, mittels denen die Endverteilerkanäle mit der flüssigen Phase (z.B. Ölfraktion bzw. Leicht- oder Schwerölfraktion) gespeist werden, wobei sich die Vorverteilerkanäle insbesondere quer zur Längsachse bzw. entlang des besagten Kolonnenquerschnitts erstrecken. Vorzugsweise verlaufen die Vorverteilerkanäle quer zu den Endverteilerkanälen. Die Vorverteilerkanäle können über zumindest einen Ausgleichskanal strömungstechnisch miteinander verbunden sein, so dass ein etwaiger Pegelunterschied der flüssigen Phase in den Vorverteilerkanälen über den mindestens einen Ausgleichskanal ausgeglichen werden kann.

Weiterhin weisen auch die Vorverteilerkanäle vorzugsweise jeweils einen quer zur Längsachse bzw. entlang des Kolonnenquerschnitts erstreckten Boden und zwei davon abgehende, längs erstreckte sowie einander gegenüber liegende Seitenwände auf, wobei die besagten Seitenwände jeweils einen oberen Rand aufweisen, wobei an jenen Rändern Auslässe in Form von insbesondere rechteckförmigen Ausnehmungen ausgebildet sind, über die die flüssige Phase in jeweils einen zugeordneten Endverteilerkanal eingeleitet wird. Vorzugsweise sind jene Auslässe der Vorverteilerkanäle jeweils lotrecht oberhalb eines zugeordneten Endverteilerkanals angeordnet. Auch hier weisen die besagten Ausnehmungen oder Ausschnitte an den Rändern jeweils eine Unterkante auf, über die die flüssige Phase aus dem jeweiligen Vorverteilerkanal in einen darunter liegenden Endverteilerkanal geführt wird, wobei jene Unterkante entlang der Längsachse des Kolonnenmantels zum jeweiligen Boden des betrachteten Vorverteilerkanals beabstandet ist, so dass der jeweilige Vorverteilerkanal wiederum bis hin zu den besagten Unterkanten seiner Auslässe Verschmutzungen ansammeln kann und dennoch die zu verteilende flüssige Phase über die Auslässe bzw. Ausnehmungen auf den jeweils zugeordneten Endverteilerkanal geben kann. Des Weiteren sind die Vorverteilerkanäle jeweils stirnseitig durch eine weitere Seitenwand begrenzt.

Vorzugsweise werden die besagten Vorverteilerkanäle über zumindest je ein Einspeiserohr, vorzugsweise über je zwei Einspeiserohre mit der flüssigen Phase, d.h. mit der Ölfraktion oder der Leicht- bzw. Schwerölfraktion, beschickt, wobei sich jene Einspeiserohre zumindest abschnittsweise entlang der Längsachse des Mantels der Kolonne erstrecken, so dass ein Auslass des jeweiligen Einspeiserohres, über den die flüssige Phase aus dem jeweiligen Einspeiserohr ausgegeben wird, entlang der Längsachse dem jeweiligen Boden des zu versorgenden Vorverteilerkanales zugewandt ist. Die besagten Einspeiserohre sind im Bereich ihres jeweiligen Auslasses vorzugsweise je zwischen zwei parallel zueinander verlaufenden Spritzblechen angeordnet, die jeweils an einer zugeordneten Seitenwand des jeweiligen Vorverteilerkanals festgelegt sind. Weiterhin weisen die Vorverteilerkanäle an den nach außen gewandten Außenseiten ihrer Seitenwände zu beiden Seiten der Auslässe des jeweiligen Vorverteilerkanals je ein Leitblech auf, wobei die Leitbleche vorzugsweise senkrecht von der jeweiligen Seitenwand abstehen und jeweils mit einem unteren freien Endbereich in den unterhalb des jeweiligen Auslasses angeordneten Endverteilerkanal hinein stehen. Die Leitbleche sind dazu ausgebildet, den Strom der flüssigen Phase aus den Auslässen der Vorverteilerkanäle derart zu leiten, dass dieser jeweils möglichst vollständig im zugeordneten Endverteilerkanal landet.

Die Vorverteiler- und Endverteilerkanäle sind also in der vorstehend beschriebenen Ausführungsform vorzugsweise als nach oben hin offene Kanäle konzipiert (nach unten hin werden die Kanäle durch die besagten Böden, zu den Seiten hin durch die besagten Seitenwände sowie die stirnseitigen weiteren Seitenwände begrenzt). Die besagten Kanäle werden daher auch als Vorverteiler- bzw. Endverteilerrinnen bezeichnet.

Die zweiten Stoffaustauschböden des Ölabschnitts bzw. des Leichtöl- und des Schwerölabschnitts ermöglichen mit Vorteil die Erzeugung einer Vielzahl von Vorhängen aus der flüssiger Phase (z.B. Ölfraktion), nämlich durch die von den Ablaufflächen abfließende flüssige Phase, sowie ggf. zusätzlich die Erzeugung einer Zweiphasenschicht auf den Ablaufflächen, was zu einem guten Wirkungsgrad beiträgt. Diesbezüglich hat sich in Versuchen gezeigt, dass die erfindungsgemäßen Stoffaustauschböden, die auch als Kaskadenböden bezeichnet werden, mit Dual-Flow-Böden vergleichbar sind (hierbei handelt es sich um Kolonnenböden ohne Ablaufschacht mit vergleichsweise großen Durchgangsöffnungen, z.B. mit einem Durchmesser im Bereich von 20mm bis 40mm, durch die Gas und Flüssigkeit im Gegenstrom strömen). Weiterhin hat sich in Versuchen gezeigt, dass die besagten Kaskadenböden bzw. erfindungsgemäßen zweiten Stoffaustauschböden hinsichtlich der Kapazität den einflutigen side-to-side baffles überlegen sind.

Aufgrund ihres Aufbaus sind die erfindungsgemäßen zweiten Stoffaustauschböden mit Vorteil verschmutzungsunanfällig, da bspw. keine kleinflächigen Öffnungen vorhanden sind, die schnell zuwachsen könnten. Der Wirkungsgrad ist relativ hoch, und zwar gemäß Versuchen etwa doppelt so hoch wie der von ein- oder zweiflutigen side-to-side baffles. Des Weiteren übersteigt ihre Kapazität die der side-to-side baffles. Gleichfalls weisen die erfindungsgemäßen Stoffaustauschböden aufgrund der winkligen Ablaufelemente eine vergleichsweise hohe strukturelle Festigkeit auf.

Bei Ablaufelementen in Form von (insbesondere gleichschenkligen) Winkelprofilen weisen die Ablaufelemente zwei Schenkel auf, die entlang der Längsachse in Richtung auf einen darüber angeordneten Flüssigkeitsverteiler winklig aufeinander zu laufen und unter Ausbildung einer entlang des Kolonnenquerschnitts bzw. quer zur Längsachse erstreckten Kante aufeinandertreffen. Die Ablaufflächen des jeweiligen Ablaufelementes bzw. Winkelprofils werden dann durch die nach oben gewandten (d.h., dem Flüssigkeitsverteiler zugewandten) Oberseiten der Schenkel gebildet.

Vorzugsweise schließen die Schenkel bzw. Ablaufflächen einen Winkel im Bereich von 80° bis 100° ein, insbesondere einen Winkel von 90°. Des Weiteren weisen die Ablaufflächen eine Breite quer zu Ihrer Längserstreckungsrichtung auf, die im Bereich von 40mm bis 150mm, vorzugsweise 100mm, liegt.

Die Ablaufelemente bzw. deren Ablaufflächen (oder die besagten Schenkel) sind bevorzugt längserstreckt ausgebildet, d.h., sie weisen entlang ihrer Längserstreckungsrichtung eine größere Länge auf als quer zu dieser Richtung, und erstrecken sich bevorzugt über den gesamten Kolonnenquerschnitt, d.h., von einem Innenseitenbereich des Mantels hin zu einem gegenüberliegenden Innenseitenbereich des Mantels der Ölwaschkolonne. Die Ablaufelemente können dabei aus mehreren Segmenten, die jeweils für sich wie ein Ablaufelement ausgebildet sind und die entlang der Längserstreckungsrichtung hintereinander angeordnet sind, zusammengesetzt sein. Ein zwischen zwei solchen Segmenten vorhandener Spalt (der die Ablaufflächen des zusammengesetzten Ablaufelementes unterbricht) kann durch ein Kappenelement, das an den beiden Ablaufflächensegmenten des jeweiligen Segments anliegt, verdeckt werden, so dass sich insgesamt ein einheitlich anmutendes Ablaufelement ergibt, das sich insbesondere im Wesentlichen entlang des gesamten Kolonnenquerschnitts von einem Innenseitenbereich des Mantels der Ölwaschkolonne zu einem gegenüberliegenden Innenseitenbereich des Mantels der Ölwaschkolonne erstreckt.

Die zweiten Stoffaustauschböden erstrecken sich jeweils vorzugsweise über den gesamten Kolonnenquerschnitt quer zur Längsachse, wobei die parallel zueinander verlaufenden Ablaufelemente vorzugsweise quer zu Ihrer Längserstreckungsrichtung äquidistant zueinander angeordnet sind, so dass je zwei benachbarte Ablaufelemente eine längliche Durchgangsöffnung oder Lücke des Stoffaustauschbodens definieren, durch die hindurch eine gasförmige Phase (z.B. ein Spaltgasstrom) in der Kolonne entlang der Längsachse des Mantels der Kolonne aufsteigen kann. Benachbarte Ablaufelemente eines zweiten Stoffaustauschbodens weisen dabei bevorzugt einen Abstand ihrer Kanten im Bereich von 150mm bis 300mm auf. Der Abstand kann jedoch auch hiervon abweichende Werte annehmen. Der besagte Abstand ist jeweils so bemessen, dass die von den Ablaufelementen abfließende flüssige Phase auf die darunter angeordneten Ablaufelemente auftrifft.

Die zweiten Stoffaustauschböden sind vorzugsweise so übereinander angeordnet, dass die Ablaufelemente zweier benachbarter zweiter Stoffaustauschböden versetzt zueinander angeordnet sind, wobei die Ablaufelemente des jeweils unteren zweiten Stoffaustauschbodens jeweils entlang der Längsachse mittig unter einer z.B. durch zwei benachbarte Ablaufelemente des jeweils oberen zweiten Stoffaustauschbodens begrenzten Durchgangsöffnung angeordnet sind.

Die zweiten Stoffaustauschböden weisen weiterhin bevorzugt einen Tragring auf, über den der jeweilige zweite Stoffaustauschboden insbesondere am Mantel festgelegt ist, wobei der Tragring vorzugsweise an einer Innenseite des Mantels der Ölwaschkolonne entlang des besagten Kolonnenquerschnitts umläuft. Die Ablaufelemente liegen dabei bevorzugt jeweils mit einem ersten und einem gegenüberliegenden zweiten Endbereich auf dem Tragring auf. Hierbei ist einer der Endbereiche über ein Festlager auf dem Tragring gelagert, der andere Endbereich über ein Gleitlager. Besteht ein Ablaufelement aus mehreren Segmenten, so ist pro Segment ein Festlager vorgesehen, die anderen Lager des betreffenden Segmentes sind Gleitlager.

Des Weiteren können die Ablaufelemente durch einen, zwei oder mehrere parallel zueinander verlaufende, entlang des besagten Kolonnenquerschnitts erstreckte Träger (insbesondere Profilträgern) unterstützt sein, die quer zu den Ablaufelementen verlaufen und auf denen die Ablaufelemente bzw. deren Komponenten (siehe oben) aufliegen. Derartige Träger greifen dabei mit Ihren einander gegenüberliegenden Endbereichen vorzugsweise jeweils unter den Tragring und sind dabei jeweils auf einer Seite über ein unterhalb des Tragrings angeordnetes Gleitlager mit dem Mantel verbunden und auf der anderen Seite über ein unterhalb des Tragrings angeordnetes Festlager.

Weitere Einzelheiten und Vorteile der Erfindung sollen durch die nachfolgenden Figurenbeschreibungen von Ausführungsbeispielen anhand der Figuren erläutert werden.

Es zeigen:
- Fig. 1: eine schematische Schnittansicht einer Ölwaschkolonne aus dem Stand der Technik;
- Fig. 2: eine schematische Schnittansicht einer Ölwaschkolonne zur Durchführung des erfindungsgemäßen Verfahrens;
- Fig. 3: ein Detail der Figur 2;
- Fig. 4: das Detail IV gemäß Figur 3;
- Fig. 5: eine Draufsicht auf das Detail gemäß Figur 4;
- Fig. 6: eine teilweise geschnittene Ansicht entlang der Linie VI-VI der Figur 5;
- Fig. 7: eine Detailansicht eines Festlagers eines erfindungsgemäßen Ablaufelementes;
- Fig. 8: eine ausschnitthafte Schnittansicht des Details VIII gemäß Figur 3;
- Fig. 9: eine Draufsicht auf einen erfindungsgemäßen Stoffaustauschboden (unterer Teil) sowie auf einen Flüssigkeitsverteiler (oberer Teil) zum Beaufschlagen des Stoffaustauschbodens mit einer flüssigen Phase;
- Fig. 10: eine ausschnitthafte Ansicht entlang der Richtung X der Figur 9 auf einen Ausgleichskanal des Flüssigkeitsverteilers, der zwei Vorverteilerkanäle des Flüssigkeitsverteilers miteinander verbindet;
- Fig. 11: eine ausschnitthafte Ansicht entlang der Richtung XI der Figur 9 auf einen Endverteilerkanal mit Auslässen in Form von rechteckförmigen Ausnehmungen, die entlang eines Randes einer Seitenwand des Endverteilerkanals ausgebildet sind;
- Fig. 12: eine ausschnitthafte Ansicht entlang der Richtung XII der Figur 9 auf einen Vorverteilerkanal mit Auslässen in Form von rechteckförmigen Ausnehmungen, die entlang eines Randes einer Seitenwand des Vorverteilerkanals ausgebildet sind;
- Fig. 13: eine ausschnitthafte, teilweise geschnittene Ansicht eines Einspeiserohres für einen Vorverteilerkanal eines Flüssigkeitsverteilers gemäß Figuren 9 bis 12;
- Fig. 14: eine weitere ausschnitthafte, teilweise geschnittene Ansicht eines Einspeiserohres für einen Vorverteilerkanal eines Flüssigkeitsverteilers gemäß Figuren 9 bis 13;
- Fig. 15 bis 17: schematische Schnittdarstellungen von Sieb, Glocken, bzw. Ventilböden.

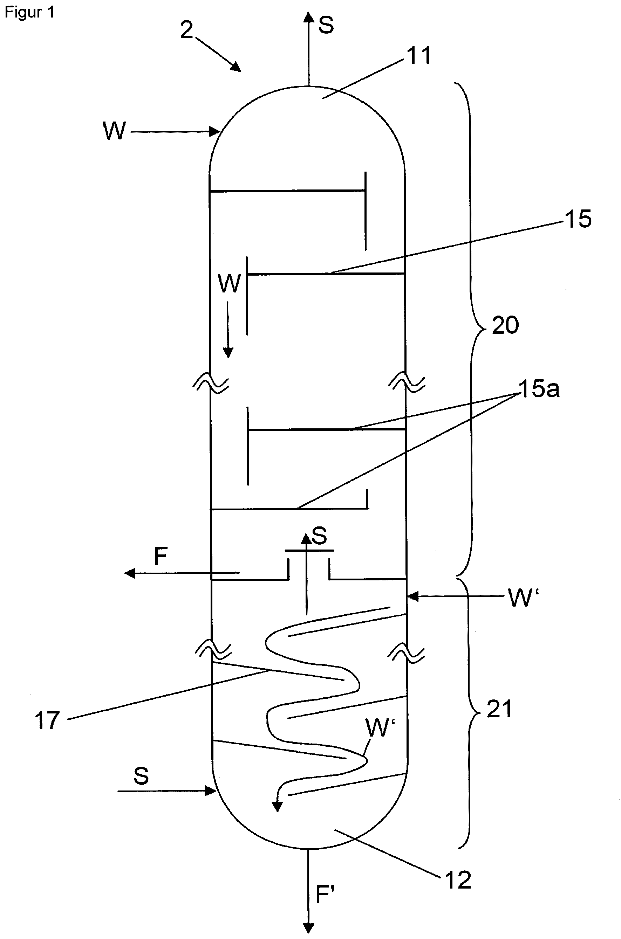

Figur 1 zeigt eine Ölwaschkolonne 2 aus dem Stand der Technik. Die Ölwaschkolonne 2 weist einen oberen Benzinabschnitt 20 und einen unteren Ölabschnitt 21 auf, wobei in einen unteren Bereich des Ölabschnitts 21 ein Spaltgasstrom S eingeleitet wird, der durch Dampfspaltung eines kohlenwasserstoffhaltigen Einsatzes (z.B. Naphtha) erzeugt wurde (siehe oben). Der Spaltgasstrom S wird im Ölabschnitt 21 im Gegenstrom mit einem flüssigen, kohlenwasserstoffhaltigen zweiten Waschmittel W' beaufschlagt, das im Ölabschnitt 21 auf zweite Stoffaustauschböden in Form von einflutigen side-to-side baffles 17 gegeben wird. Hierdurch werden schwerer-siedende Kohlenwasserstoffe aus dem Spaltgasstrom S abgetrennt und sammeln sich im Sumpf 12 als Ölfraktion F'. Im Benzinabschnitt 20 wird der Spaltgasstrom S mittels ersten Stoffaustauschböden in Form von Sieb- oder Ventilböden 15, auf die eine flüssiges, kohlenwasserstoffhaltiges erstes Waschmittel W gegeben wird, mit dem ersten Waschmittel W in Kontakt gebracht, um eine vergleichsweise leichter-siedende Benzinfraktion F aus dem Spaltgasstrom S abzutrennen.

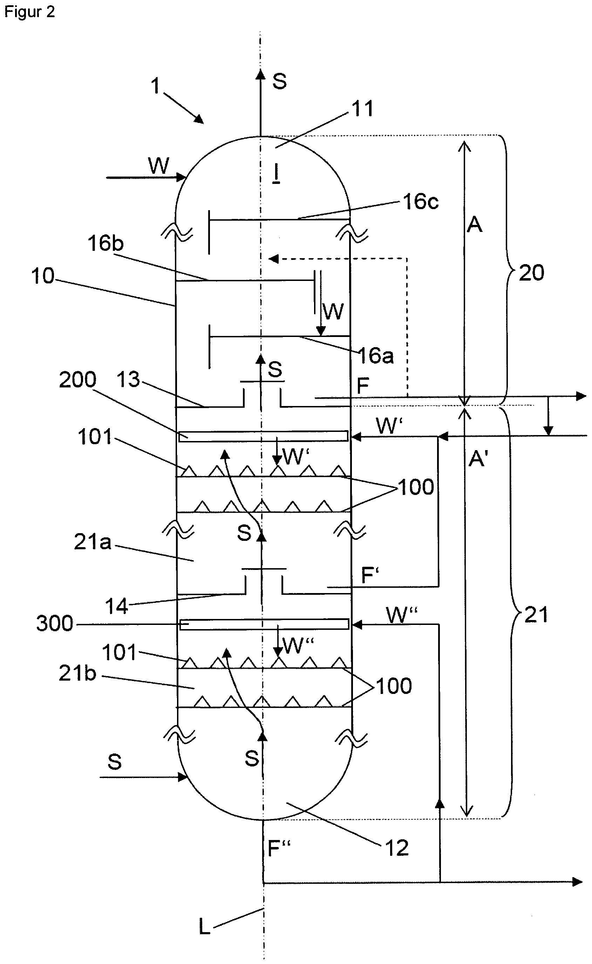

Das erfindungsgemäße Verfahren, das anhand der Ölwaschkolonne 1 gemäß Figur 2 beispielhaft erläutert wird, ist nun so konzipiert, dass einer Verschmutzung auf den unteren ersten Stoffaustauschböden 15a der Ölwaschkolonne 2 gemäß Figur 1 entgegen gewirkt wird.

Im Einzelnen weist die Ölwaschkolonne 1 gemäß Figur 2 einen im Wesentlichen zylindrischen Mantel 10 auf, der sich entlang einer mit der Vertikalen zusammenfallenden Längsachse L erstreckt und einen Innenraum der Ölwaschkolonne 1 begrenzt, der entlang der Längsachse L in einen den Kopf 11 der Ölwaschkolonne 1 aufweisenden Benzinabschnitt 20 und einen darunter angeordneten, den Sumpf 12 der Ölwaschkolonne 1 aufweisenden Ölabschnitt 21 unterteilt ist, der wiederum in einen Leichtölabschnitt 21a und einen darunter angeordneten Schwerölabschnitt 21b unterteilt sein kann. Dabei ist der Benzinabschnitt 20 durch einen Kaminboden 13 vom Ölabschnitt 21 bzw. Leichtölabschnitt 21a getrennt, und der Leichtölabschnitt 21a ist durch einen Kaminboden 14 vom Schwerölabschnitt 21b getrennt.

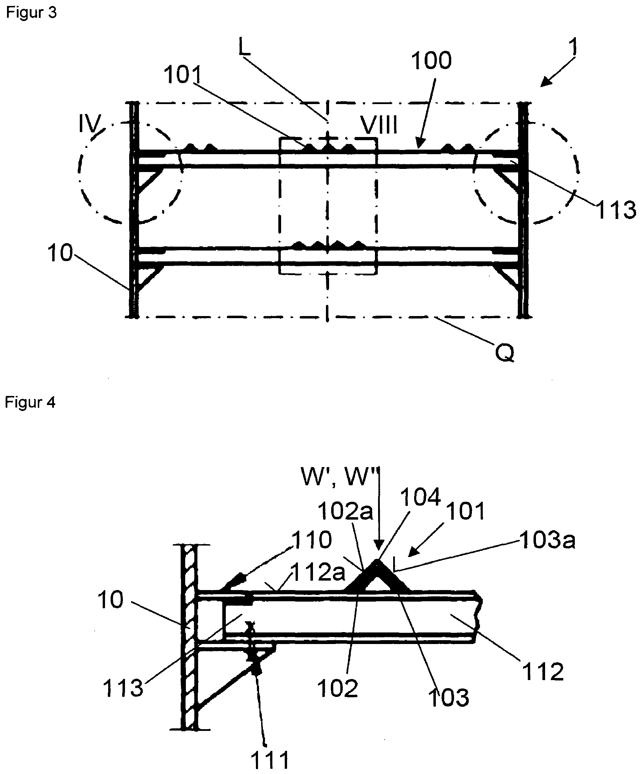

Der vorgekühlte Spaltgasstrom S wird mit einer Temperatur zwischen z.B. ca. 400°C und 600°C der Ölwaschkolonne 1 im Bodenbereich des Schwerölabschnitts 21b zugeführt. Der Schwerölabschnitt 21b der Ölwaschkolonne 1 weist dabei mehrere entlang der Längsachse L übereinander angeordnete zweite Stoffaustauschböden 100 auf, die in den Figuren 3 bis 9 gezeigt sind. Dabei sind - wie vorstehend beschrieben - jeweils benachbarte zweite Stoffaustauschböden 100 so ausgebildet, dass die Ablaufelemente 101 des jeweils unteren zweiten Stoffaustauschbodens 100 mittig unter den Durchgangsöffnungen bzw. Lücken des darüber angeordneten zweiten Stoffaustauschbodens 100 angeordnet sind.

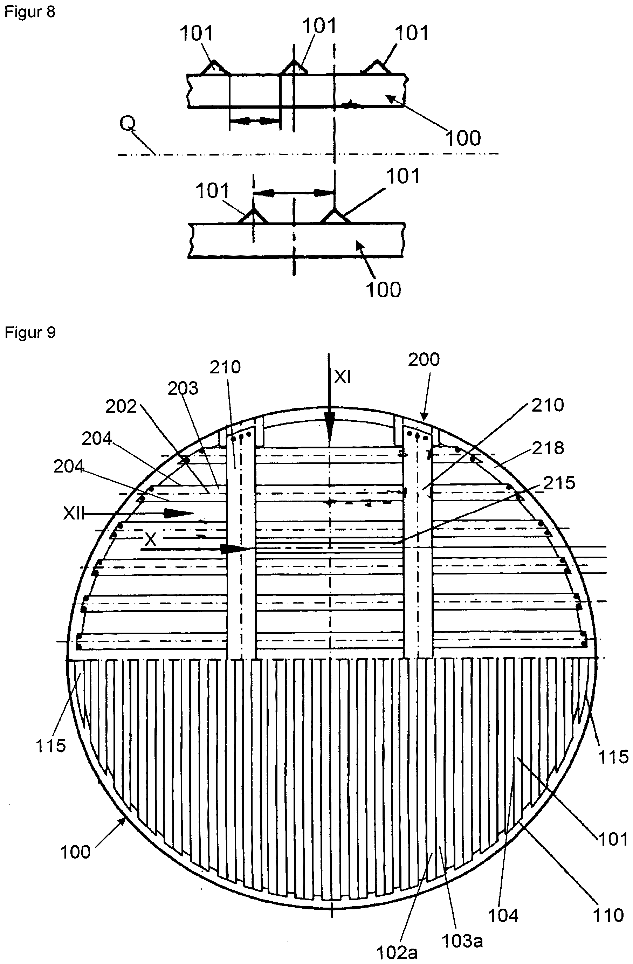

Das Spaltgas S durchströmt den gesamten Innenraum der Ölwaschkolonne 1 von unten nach oben entlang der Längsachse L des Mantels 10 der Kolonne 1, wobei im Schwerölabschnitt 21b ein flüssiges, kohlenwasserstoffhaltiges drittes Waschmittel W" mittels eines in den Figuren 9 bis 14 gezeigten zweiten Flüssigkeitsverteilers 300, der entlang der Längsachse L oberhalb der zweiten Stoffaustauschböden 100 angeordnet ist, auf die zweiten Stoffaustauschböden 100 aufgegeben wird. Das dritte Waschmittel W" strömt entsprechend im Schwerölabschnitt 21b herab und wird durch die zweiten Stoffaustauschböden 100 in intensiven Kontakt mit dem aufsteigenden Spaltgasstrom S gebracht. Dadurch wird aus dem Spaltgasstrom S der Anteil an den schwersten Kohlenwasserstoffen abgetrennt, wobei sich jene Kohlenwasserstoffe als flüssige Schwerölfraktion F' im Sumpf 12 der Ölwaschkolonne 1 sammeln. Die Schwerölfraktion F" wird von dort abgezogen, gekühlt und als drittes Waschmittel W" zumindest teilweise in den Schwerölabschnitt 21b zurückgeführt und erneut mittels des zweiten Flüssigkeitsverteilers 300 auf einen obersten zweiten Stoffaustauschboden 100 bzw. auf die Spaltgasphase S aufgegeben.

Aus dem Schwerölabschnitt 21b gelangt der an der Schwerölfraktion abgereicherte Spaltgasstrom S über den Kaminboden 14 in den Leichtölabschnitt 21a der Ölwaschkolonne 1. Hier steigt die Spaltgasphase S ebenfalls durch zweite Stoffaustauschböden 100 weiter nach oben auf, die im Gegenstrom über einen ersten Flüssigkeitsverteiler 200 (siehe Figuren 9 bis 14) des Leichtölabschnitts 21a mit einem flüssigen, kohlenwasserstoffhaltigen zweiten Waschmittel W' beaufschlagt werden, so dass sich entsprechende, aus der Gasphase S abgetrennte, leichter-siedende Kohlenwasserstoffe als flüssige Leichtölfraktion im Leichtölabschnitt 21a, insbesondere auf dem Kaminboden 14, sammeln. Von dort aus kann jene Leichtölfraktion ggf. über Abläufe direkt in die Vorverteilerkanäle 210 des zweiten Flüssigkeitsverteilers 300 des Schwerölabschnitts 21b gelangen. Weiterhin wird die besagte Leichtölfraktion F' aus dem Leichtölabschnitt 21a abgezogen, gekühlt und mit einer aus dem Benzinabschnitt 20 abgezogenen Benzinfraktion F vermischt und als zweites Waschmittel W' über den besagten ersten Flüssigkeitsverteiler 200 in den Leichtölabschnitt 21a zurückgeführt.

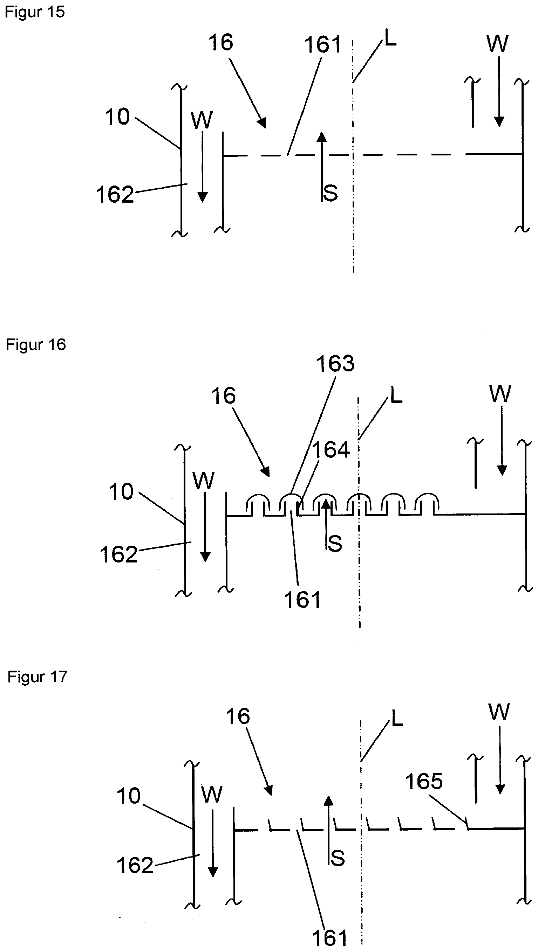

Aus dem Leichtölabschnitt 21a gelangt der an der Leichtölfraktion F' abgereicherte Spaltgasstrom S über den Kaminboden 13 schließlich in den Benzinabschnitt 20 der Ölwaschkolonne 1, wo der Spaltgasstrom S über erste Stoffaustauschböden 16a, 16b, 16c in Form von Sieb-, Glocken- oder Ventilböden (vgl. Figuren 15 bis 17) in den Kopf 11 der Kolonne 1 gelangt und von dort abgezogen wird. Im Benzinabschnitt 20 wird der Spaltgasstrom S mit einem flüssigen, kohlenwasserstoffhaltigen ersten Waschmittel W in Kontakt gebracht, das auf die ersten Stoffaustauschböden 16a, 16b, 16c aufgegeben wird, so dass leichter-siedende Kohlenwasserstoffe aus dem Spaltgasstrom S abgetrennt werden und sich im Benzinabschnitt 20 als flüssige Benzinfraktion F sammeln. Die Benzinfraktion F wird aus dem Benzinabschnitt 20 abgezogen und teilweise mit der aus dem Leichtölabschnitt 21a abgezogenen Leichtölfraktion F' gemischt und in den Leichtölabschnitt 21a als zweites Waschmittel W' zurückgeführt (siehe oben). Weiterhin kann ein Teil der Benzinfraktion F auf einen der unteren ersten Stoffaustauschböden zurückgeführt werden (so genannter pump-around), z.B. auf den zweituntersten ersten Stoffaustauschboden 16b von unten, um die Zirkulation auf den unteren ersten Stoffaustauschböden 16a, 16b zu erhöhen, was einer Verlegung der besonders verschmutzungsanfälligen unteren ersten Stoffaustauschböden 16a, 16b durch Polymerbildung entgegenwirkt (siehe oben).

Weiterhin wird erfindungsgemäß eine Benzinfraktion F als Waschmittel W bzw. als Bestandteil des Waschmittels W in den Kopf 11 der Ölwaschkolonne 1 bzw. des Benzinabschnitts 20 zurückgeführt, wobei bevorzugt das Verhältnis zwischen diesem Benzinrücklauf (kmol/h) und der in den Ölabschnitt 21 bzw. Schwerölabschnitt 21b eingespeisten Spaltgasstoffmenge S (kmol/h) zwischen 1:16 und 1:10 liegt, bevorzugt zwischen 1:12 bis 1:10. Die in den Kopf 11 zurückgeführte Benzinfraktion (so genannter Rücklauf) stammt dabei bevorzugt aus einer der Ölwaschkolonne 1 nachfolgenden Wasserwaschkolonne, aus deren Sumpf sie in einen Benzin-Wasser-Trennbehälter gelangt, von dem aus sie vorzugsweise wasserfrei als Rücklauf in den Kopf 11 der Ölwaschkolonne 1 gegeben wird. Beim Anfahren der Anlage kann zusätzlich Fremdbenzin in den Kopf 11 der Ölwaschkolonne 1 gegeben werden (siehe oben).

Bevorzugt weist der Benzinabschnitt 20 der Ölwaschkolonne 1 entlang der Längsachse L eine Länge A auf, die kleiner ist als die Länge A' des Ölabschnitts 21, vorzugsweise kleiner als die Hälfte der Länge A' des Ölabschnitts 21.

Für den Fall, dass das Spaltgas S durch Dampfspaltung eines Einsatzes wie Naphtha oder eines leichteren Einsatzes erzeugt wird, kann der Schwerölabschnitt 21b entfallen. Aus dem Sumpf 12 des Ölabschnitts 21 wird dann eine Ölfraktion F' abgezogen, die analog zur Leichtölfraktion F' im obigen Ausführungsbeispiel behandelt wird.

Die Figuren 3 bis 9 zeigen die zweiten Stoffaustauschböden 100 im Detail. Allgemein können derartige Stoffaustauschböden 100 überall dort in Kolonnen mit Vorteil eingesetzt werden, wo aufgrund der in Stoffaustausch zu bringenden gasförmigen bzw. flüssigen Phasen ein hohes Verschmutzungsrisiko herrscht.

Die zweiten Stoffaustauschböden 100 weisen gemäß Figur 9 eine Mehrzahl an längs erstreckten, parallel zueinander orientierten Ablaufelementen 101 auf, die sich parallel zum senkrecht zur Längsachse L verlaufenden Kolonnenquerschnitt Q auf gleicher Höhe (bezogen auf die Längsachse L des Mantels 10) erstrecken. Benachbarte Ablaufelemente 101 sind dabei quer zu ihrer Längserstreckungsrichtung äquidistant zueinander beabstandet, so dass zwischen je zwei Ablaufelementen 101 eine Durchgangsöffnung oder Lücke gebildet wird, durch die gasförmige Phase S im Innenraum der Ölwaschkolonne 1 aufsteigen kann.

Die Ablaufelemente 101 weisen gemäß den Figuren 4 bis 7 und 9 jeweils einen ersten und einen zweiten Schenkel 102, 103 auf, die winklig miteinander unter Ausbildung einer Kante 104 verbunden sind, so dass die Ablaufelemente 101 geichschenklige Winkelprofile 101 bilden. Dabei sind die jeweiligen Kanten 104 der Ablaufelemente 101 ebenfalls längs erstreckt ausgebildet und verlaufen parallel zum Kolonnenquerschnitt Q. Weiterhin laufen die Schenkel 102, 103 der Ablaufelementes 101 entlang der Längsachse L nach oben hin aufeinander zu, so dass die beiden Schenkel 102, 103 eines Ablaufelementes 101 je eine nach oben gewandte Ablauffläche 102a, 103a definieren, die jeweils ausgehend von der Kante 104 des jeweiligen Ablaufelementes 101 nach unten hin abfallen. Wird entsprechend das jeweilige flüssige Waschmittel W', W" durch einen ersten oder zweiten Flüssigkeitsverteiler 200, 300 auf die jeweilige Kante 104 eines Ablaufelementes 101 aufgegeben, fließt dieses zu beiden Seiten der jeweiligen Kante 104 über die Ablaufflächen 102a, 103a nach unten ab, so dass je zwei Vorhänge des betreffenden Waschmittels W', W" gebildet werden.

Gemäß Figur 8 sind mehrere erfindungsgemäße zweite Stoffaustauschböden 100 entlang der Längsachse L übereinander angeordnet, wobei die Ablaufelemente 101 benachbarter zweiter Stoffaustauschböden 100 entlang des Kolonnenquerschnitts Q versetzt zueinander angeordnet sind, so dass die von den Ablaufflächen 102a, 103a des jeweiligen Ablaufelementes 101 eines zweiten Stoffaustauschbodens 100 ablaufende flüssige Phase W, W" auf zwei unterhalb jenes Ablaufelementes 101 angeordnete Ablaufelemente 101 eines darunter liegenden zweiten Stoffaustauschbodens 100 aufgeben wird. Dabei sind die Ablaufelemente 101 des jeweils unteren zweiten Stoffaustauschbodens 100 entlang des Kolonnenquerschnitts Q bevorzugt jeweils mittig zwischen zwei Ablaufelementen 101 des darüber befindlichen zweiten Stoffaustauschbodens 100 angeordnet. Die erfindungsgemäßen zweiten Stoffaustauschböden 100 werden daher auch Kaskadenböden genannt.

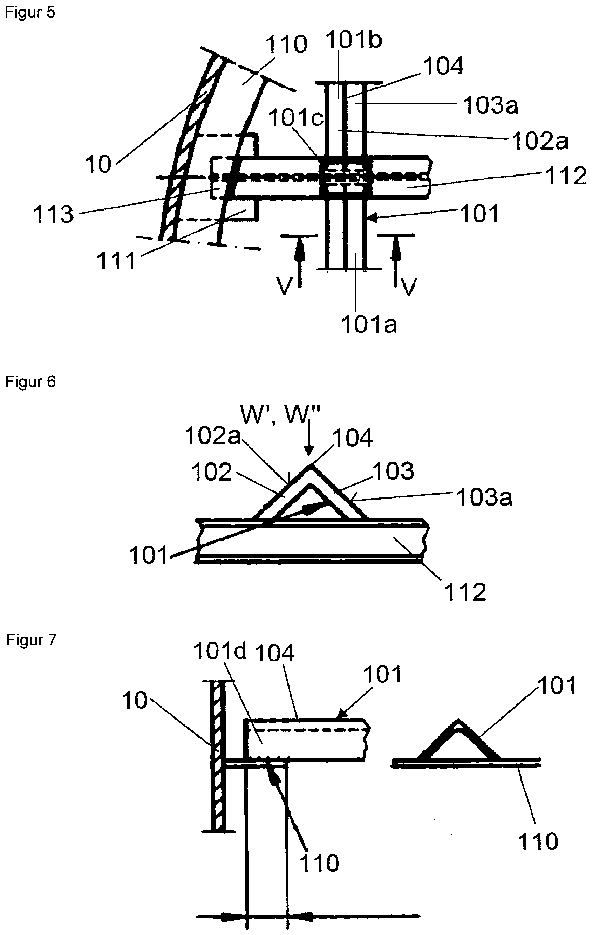

Gemäß den Figuren 4 - 7 liegen die Ablaufelemente 101 eines zweiten Stoffaustauschbodens 100 mit einander gegenüberliegenden Endbereichen 101d (vgl. Fig. 7) auf einem zugeordneten, umlaufenden Tragring 110 auf, der an einer Innenseite des Mantels 10 der Ölwaschkolonne 1 festgelegt ist. Dabei ist jeweils ein Endbereich 101d über ein Festlager, der andere über ein Gleitlager auf dem Tragring 110 gelagert.

Die Ablaufelemente 101 können sich durchgehend über den Kolonnenquerschnitt Q von einem Innenseitenbereich des Mantels 10 der Ölwaschkolonne 1 zu einem gegenüberliegenden Innenseitenbereich des Mantels 10 der Ölwaschkolonne 1 erstrecken. Es besteht aber auch die Möglichkeit, dass ein Ablaufelement 101 aus mehreren Segmenten 101a, 101b besteht (vgl. Fig. 5), die entlang der Längserstreckungsrichtung des Ablaufelementes 101 hintereinander angeordnet sind. Dabei können Spalte zwischen zwei benachbarten Segmenten 101a, 101b mit einer Kappe 101c überdeckt sein. Derartige Segmente 101a, 101b liegen dann mit ihren freien Endbereichen auf dem Tragring 110 und/oder auf einem Träger 112, insbesondere Profilträger 112, auf, der sich quer zu den Ablaufelementen 101 erstreckt. Ggf. können mehrere derartige Träger 112 vorgesehen werden, die dann parallel zu einander verlaufen. Ein Endbereich eines Segmentes 101a, 101b ist dann über ein Festlager auf dem Tragring 110 oder einem Träger 112 gelagert, der jeweils andere Endbereich über ein Gleitlager.

Die Träger 112, sofern vorhanden, untergreifen den Tragring 110 mit je einem freien Endbereich 113, der auf einem unterhalb des jeweiligen Tragrings 110 an der Innenseite des Mantels 10 festgelegten Lager 111 aufliegt. Die Endbereiche 113 des jeweiligen Trägers 112 weisen dabei eine Ausnehmung zur Aufnahme des zugeordneten Tragrings 110 auf, so dass der jeweilige Tragring 110 zusammen mit dem jeweiligen Träger 112 eine im Wesentliche stufenlose Oberfläche 112a ausbildet, auf der die Ablaufelemente 101 aufliegen können (vgl. Fig. 4). Bei den Trägern 112 ist jeweils ebenfalls bevorzugt ein Endbereich 113 über ein Lager 111 in Form eines Gleitlagers 111 (vgl. Fig. 4) am Mantel 10 gelagert, wohingegen der andere Endbereich 113 über ein Festlager gelagert ist (vgl. Fig. 3).

Weiterhin können die zweiten Stoffaustauschböden 100 gemäß Figur 9 jeweils seitlich eines äußersten Ablaufelementes 101 ein Abdeckelement 115 aufweisen, das dazu dient, die Durchgangsöffnung zwischen dem besagten Ablaufelement 101 und dem Abdeckelement 115 auf die vorgesehene Breite zu begrenzen.

Zum Beaufschlagen der übereinander angeordneten zweiten Stoffaustauschböden 100 mit dem jeweiligen flüssigen Waschmittel W', W" ist gemäß den Figuren 2 sowie 9-14 ein erster bzw. zweiter Flüssigkeitsverteiler 200, 300 vorgesehen. Diese weisen jeweils mehrere nach oben hin offene, im Querschnitt kastenförmige Endverteilerkanäle 202 auf, die entlang der Längsachse L der Ölwaschkolonne 1 oberhalb der jeweils zugeordneten zweiten Stoffaustauschböden 100 angeordnet sind und sich jeweils entlang des besagten Kolonnenquerschnitts Q sowie quer zu den Ablaufelementen 101 erstrecken.

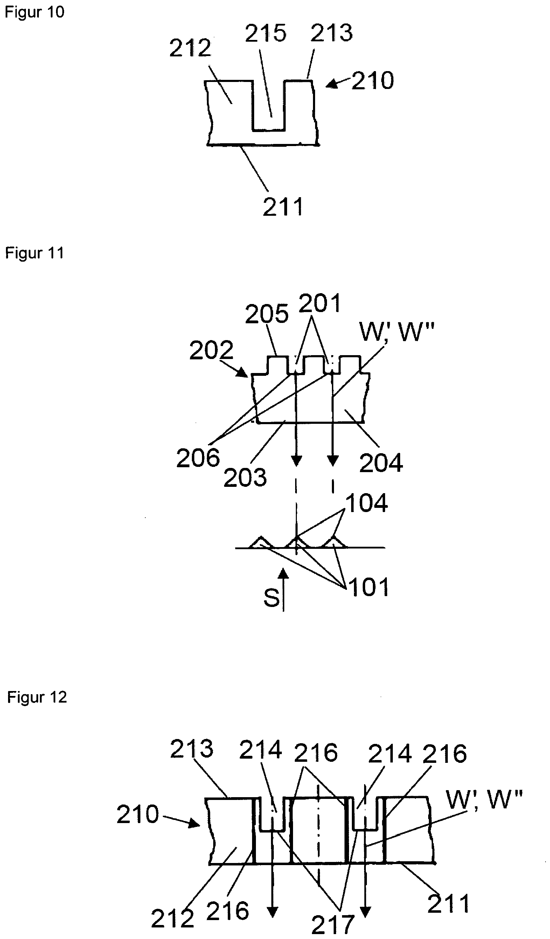

Die längs erstreckten Endverteilerkanäle 202 weisen jeweils einen parallel zum Kolonnenquerschnitt Q erstreckten Boden 203 auf sowie zwei davon abgehende Seitenwände 204, wobei die besagten Seitenwände 204 jeweils einen oberen Rand 205 aufweisen (vgl. Fig. 11), an dem entlang Auslässe 201 in Form von rechteckförmigen Ausnehmungen ausgebildet sind, die entlang der Längsachse L der jeweiligen Kolonne 1, 3, 4 jeweils lotrecht oberhalb einer Kante 104 eines dem jeweiligen Auslass 201 zugeordneten Ablaufelementes 101 angeordnet sind. Die Endverteilerkanäle 202 werden zum Verteilen der flüssigen Phase W', W" auf die Ablaufelemente 101 nun so mit der flüssigen Phase W', W" beschickt, das diese über die parallel zum jeweiligen Boden 203 verlaufenden Unterkanten 206 der einzelnen Auslässe 201 tritt und auf die besagten Kanten 104 herabfällt und durch die kaskadierten Ablaufelemente 101 (vgl. Fig. 8) weiter nach unten verteilt wird, so dass sich eine Vielzahl an Vorhängen der flüssigen Phase W', W" bilden, durch die eine zu behandelnde gasförmige Phase (z.B. Spaltgas) S im Gegenstrom zwangsgeführt wird, so dass sich ein intensiver Stoff- und/oder Energieaustausch zwischen den Phasen W', W", S ereignet.

Zum Beschicken der Endverteilerkanäle 202 mit der flüssigen Phase W', W" sind gemäß Figur 10 zwei zueinander parallele, entlang der Längsachse L oberhalb der Endverteilerkanäle 202 angeordnete Vorverteilerkanäle 210 vorgesehen, die ebenfalls nach oben hin offen ausgebildet sind und im Querschnitt kastenförmig ausgeformt sind. Die Vorverteilerkanäle 210 erstrecken sich ebenfalls entlang des Kolonnenquerschnitts Q, und zwar vorzugsweise ebenso wie die Endverteilerkanäle 202 im Wesentlichen über den gesamten Kolonnenquerschnitt, d.h., von einem Innenseitenbereich des Mantels 10 der jeweiligen Kolonne 1, 3, 4 hin zu einem gegenüberliegenden Innenseitenbereich des Mantels 10. Des Weiteren erstrecken sich die Vorverteilerkanäle 210 quer zu den Endverteilerkanälen 202.

Die Vorverteilerkanäle 210 weisen ebenfalls jeweils einen parallel zum Kolonnenquerschnitt Q erstreckten Boden 211 auf sowie zwei davon abgehende Seitenwände 212, die jeweils einen oberen Rand 213 aufweisen, an denen Auslässe 214 in Form von rechteckförmigen Ausnehmungen ausgebildet sind, über die die flüssige Phase W', W" in jeweils einen zugeordneten Endverteilerkanal 202 einleitbar ist. Hierzu sind die Auslässe 214 der Vorverteilerkanäle 210 wiederum jeweils lotrecht entlang der Längsachse L des Mantels 10 der jeweiligen Kolonne 1, 3, 4 oberhalb eines zugeordneten Endverteilerkanals 202 angeordnet (vgl. Figuren 12, 13 und 14).

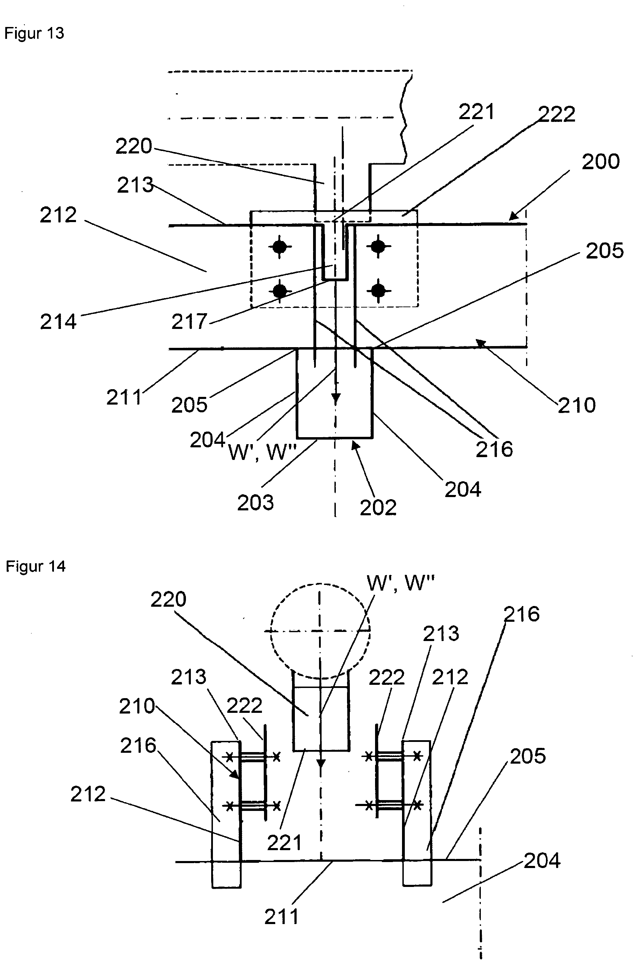

Die besagten Vorverteilerkanäle 210 werden gemäß Figuren 13 und 14 ihrerseits über zumindest je ein Einspeiserohr 220, vorzugsweise über je zwei Einspeiserohre 220 mit der flüssigen Phase F beschickt, die sich zumindest abschnittsweise entlang der Längsachse L des Mantels 10 der jeweiligen Kolonne 1, 3, 4 erstrecken, wobei ein Auslass 221 des jeweiligen Einspeiserohres 220, über den die flüssige Phase W', W" aus dem jeweiligen Einspeiserohr 220 in den zugeordneten Vorverteilerkanal 210 strömt, entlang der besagten Längsachse L dem jeweiligen Boden 211 des zu versorgenden Vorverteilerkanales 210 zugewandt ist. Die besagten Einspeiserohre 220 sind je zwischen zwei parallel zueinander verlaufenden Spritzblechen 222 angeordnet, die den Auslass 221 des jeweiligen Einspeiserohres 220 beidseitig flankieren und jeweils an einer zugeordneten Seitenwand 212 des betreffenden Vorverteilerkanals 210 festgelegt sind.

Weiterhin weisen die Vorverteilerkanäle 210 an den nach außen gewandten Außenseiten ihrer Seitenwände 212 zu beiden Seiten der Auslässe 214 des jeweiligen Vorverteilerkanals 210 je ein Leitblech 216 auf, die senkrecht von der jeweiligen Seitenwand 212 abstehen und jeweils mit einem unteren freien Endbereich in den unterhalb des jeweiligen Auslasses 214 angeordneten Endverteilerkanal 202 hinein ragen. Die Leitbleche 216 dienen dazu, den Strom der flüssigen Phase W', W" aus den Auslässen 214 der Vorverteilerkanäle 210 in die zugeordneten Endverteilerkanäle 202 zu leiten.

Damit die flüssige Phase W, W" in den beiden Vorverteilerkanälen 210 immer den gleichen Pegel aufweist, können die beiden Vorverteilerkanäle 210 gemäß Figuren 9 und 10 über zumindest einen Ausgleichskanal 215 verbunden sein, der sich zwischen den beiden Vorverteilerkanälen 210 erstreckt, und zwar quer zu diesen.

Die oben erwähnten ersten Stoffaustauschböden 16a, 16b, 16c des Benzinabschnitts 20 der Ölwaschkolonne 1 sind in den Figuren 15 bis 17 schematisch im Schnitt gezeigt. Dabei handelt es sich gemäß Figur 15 bei Siebböden 16 um Kolonnenböden mit einem Ablaufschacht 162, über den das Waschmittel W auf darunter befindliche Kolonnenböden gelangt. Siebböden 16 weisen dabei eine Mehrzahl an Durchgangsöffnungen 161 auf, durch die das Spaltgas S strömt und das auf dem jeweiligen Siebboden 16 befindliche Waschmittel W kontaktiert.

Bei ersten Stoffaustauschböden in Form von Glockenböden 16 ist gemäß Figur 16 ebenfalls ein Ablaufschacht 162 vorgesehen. Weiterhin sind bei Glockenböden 16 die besagten Durchgangsöffnungen 161 von Kaminhälsen 164 eingefasst, die von Hauben 163 überdacht sind, wobei insbesondere die Kaminhälse 164 in die jeweils zugeordnete Haube 163 hinein ragen.

Ventilböden 16 können gemäß Figur 17 ebenfalls einen Ablaufschacht 162 aufweisen. Des Weiteren sind bei Ventilböden 16 die besagten Durchgangsöffnungen 161 durch Ventile 165, insbesondere Klappen, verschließbar, um ein Durchregnen zu verhindern. Bei hinreichendem Spaltgasdruck werden die Ventile aufgedrückt, so dass das Spaltgas S von unten nach oben durch die Durchgangsöffnungen 161 des Ventilbodens strömen kann. Neben beweglichen Ventilen können auch so genannte Fixed Valves, d.h., feststehende Ventildeckel mit Promotoreneffekt verwendet werden.

**Bezugszeichenliste**

| | |
|---|---|
| 1 | Ölwaschkolonne |
| 2 | Ölwaschkolonne |
| 5 | Pumpe |
| 6 | Wärmetauscher |
| 10 | Mantel |
| 11 | Kopf |
| 12 | Sumpf |
| 13, 14 | Kaminboden |
| 15, 15a | Sieb oder Ventilböden |
| 16, 16a, 16b, 16c | Erste Stoffaustauschböden, z.B. Sieb-, Glocken- oder Ventilböden |
| 17 | Side-to-side baffels |
| 20, 21, 21a, 21b | Abschnitte der Kolonne |
| 100 | Zweite Stoffaustauschböden |
| 101 | Ablaufelement |
| 101a, 101b | Segmente |
| 101c | Kappe |
| 101d | Endbereich |
| 102, 103 | Schenkel |
| 102a, 103a | Ablaufflächen |
| 104 | Kante |
| 110 | Tragring |
| 111 | Lager |
| 112 | Träger |
| 112a | Oberfläche |
| 113 | Endbereich |
| 115 | Abdeckblech |
| 161 | Durchgangsöffnung |
| 162 | Ablaufschacht |
| 163 | Haube |
| 164 | Kaminhals |
| 165 | Ventil (insbesondere Klappe) |
| 200, 300 | Flüssigkeitsverteiler |
| 201 | Auslässe |
| 202 | Endverteilerkanal |
| 203 | Boden |
| 204 | Seitenwand |
| 205 | Rand |
| 206 | Unterkante |
| 210 | Vorverteilerkanal |
| 211 | Boden |
| 212 | Seitenwand |
| 213 | Rand |
| 214 | Auslass |
| 215 | Ausgleichskanal |
| 216 | Leitblech |
| 217 | Unterkante |
| 220 | Einspeiserohr |
| 221 | Auslass |
| 222 | Spritzblech |
| F, F', F" | Fraktionen |
| I | Innenraum |
| L | Längsachse |
| Q | Kolonnenquerschnitt |
| S | Spaltgasstrom |
| W, W', W" | Waschmittel |

## Patentansprüche

1. Verfahren zum Reinigen eines Spaltgasstroms in einer Ölwaschkolonne, die einen entlang einer Längsachse (L) erstreckten Mantel (10) aufweist, der einen Innenraum (I) der Kolonne (1) umschließt, wobei der Innenraum (I) in einen Benzinabschnitt (20) und einen entlang der Längsachse (L) darunter angeordneten Ölabschnitt (21) unterteilt ist, wobei ein Spaltgasstrom (S) in den Ölabschnitt (21) eingeleitet wird und entlang der Längsachse (L) von unten nach oben durch übereinander angeordnete zweite Stoffaustauschböden (100) des Ölabschnitts (21) geleitet wird, die im Gegenstrom zum Spaltgasstrom (S) mit einem flüssigen, kohlenwasserstoffhaltigen zweiten Waschmittel (W') beaufschlagt werden, um aus dem Spaltgasstrom (S) eine Ölfraktion (F') abzutrennen, wobei die zweiten Stoffaustauschböden (100) jeweils eine Mehrzahl an parallel und beabstandet zueinander verlaufenden Ablaufelementen (101), insbesondere in Form von Winkelprofilen, aufweisen, die jeweils eine erste und eine zweite Ablauffläche (102a, 103a) aufweisen, an denen entlang das zweite Waschmittel (W') nach unten abläuft und die entlang der Längsachse (L) zum Benzinabschnitt hin aufeinander zu laufen und unter Ausbildung einer quer zur Längsachse (L) erstreckten Kante (104) aufeinandertreffen, wobei der Spaltgasstrom nach dem Durchlaufen der zweiten Stoffaustauschböden (100) des Ölabschnitts (21) in den Benzinabschnitt (20) geführt wird und sodann entlang der Längsachse (L) von unten nach oben durch übereinander angeordnete erste Stoffaustauschböden (16a, 16b, 16c) des Benzinabschnitts (20) geleitet wird, die als Siebböden, Glockenböden und/oder Ventilböden ausgebildet sind und die im Gegenstrom zum Spaltgasstrom (S) mit einem flüssigen, kohlenwasserstoffhaltigen ersten Waschmittel (W) beaufschlagt werden, um aus dem Spaltgasstrom (S) eine Benzinfraktion (F) abzutrennen, wobei der Spaltgasstrom (S) aus einem Kopf (11) des Benzinabschnitts (20) abgezogen wird, wobei jene Benzinfraktion (F) aus dem Benzinabschnitt (20) abgezogen wird, und wobei in den Kopf (11) des Benzinabschnitts (20) eine Benzinfraktion (W) als erstes Waschmittel (W) zurückgeführt wird,
**dadurch gekennzeichnet,**
**dass** das molare Verhältnis der in den Benzinabschnitt (20) pro Zeiteinheit zurückgeführten Stoffmenge der Benzinfraktion (W) zu der pro Zeiteinheit in den Ölabschnitt (21) eingeleiteten Stoffmenge des Spaltgases (S) in einem Bereich von 1:16 bis 1:10, bevorzugt 1:12 bis 1:10 liegt.

2. Verfahren nach Anspruch 1, **dadurch gekennzeichnet, dass** ein Teil der aus dem Benzinabschnitt (20) abgezogenen Benzinfraktion (F) auf einen der ersten Stoffaustauschboden (16b, 16c) zurückgeführt wird, insbesondere auf einen der unteren ersten Stoffaustauschböden (16b, 16c).

3. Verfahren nach Anspruch 2, **dadurch gekennzeichnet, dass** es sich bei jenem ersten Stoffaustauschboden um den zweituntersten (16b), drittuntersten (16c) oder viertuntersten ersten Stoffaustauschboden handelt.

4. Verfahren nach einem der vorhergehenden Ansprüche, **dadurch gekennzeichnet, dass** die Benzinfraktion (F) von einem Kaminboden (13) oder von einem untersten ersten Stoffaustauschboden abgezogen wird, der den Benzinabschnitt (20) vom Ölabschnitt (21) abtrennt.

5. Verfahren nach einem der vorhergehenden Ansprüche, **dadurch gekennzeichnet, dass** der Ölabschnitt (21) in einen Leichtölabschnitt (21a) und einen entlang der Längsachse (L) darunter angeordneten Schwerölabschnitt (21b) unterteilt ist, in denen jeweils mehrere zweite Stoffaustauschböden (100) angeordnet sind, wobei insbesondere der Leichtölabschnitt (21a) vom Schwerölabschnitt (21b) mittels eines Kaminbodens (14) abgetrennt ist, und wobei der Spaltgasstrom (S) durch die zweiten Stoffaustauschböden (100) des Schwerölabschnitts (21b) und sodann durch die zweiten Stoffaustauschböden (100) des Leichtölabschnitts (21a) geführt wird, wobei die zweiten Austauschböden (100) im Schwerölabschnitt (21b) mit einem flüssigen, kohlenwasserstoffhaltigen dritten Waschmittel (W") beaufschlagt werden, um aus dem Spaltgasstrom (S) eine Schwerölfraktion (F") abzutrennen, und wobei die zweiten Austauschböden (100) im Leichtölabschnitt (21a) mit einem flüssigen, kohlenwasserstoffhaltigen zweiten Waschmittel (W') beaufschlagt werden, um aus dem Spaltgasstrom (S) eine Leichtölfraktion (F') abzutrennen.

6. Verfahren nach einem der vorhergehenden Ansprüche, **dadurch gekennzeichnet, dass** der Benzinabschnitt (20) 6 bis 8, insbesondere 7, erste Stoffaustauschböden (16a, 16b, 16c) aufweist, wobei insbesondere benachbarte erste Stoffaustauschböden einen Abstand zueinander entlang der Längsachse (L) im Bereich von 500mm bis 900mm aufweisen.

7. Verfahren nach einem der vorhergehenden Ansprüche, **dadurch gekennzeichnet, dass** der Ölabschnitt (21) 10 bis 20, insbesondere 16, zweite Stoffaustauschböden (100) aufweist.

8. Verfahren nach Anspruch 6, **dadurch gekennzeichnet, dass** der Leichtölabschnitt (21a) 6 bis 12, insbesondere 8, zweite Stoffaustauschböden (100) aufweist,

9. Verfahren nach Anspruch 6 oder 8, **dadurch gekennzeichnet, dass** der Schwerölabschnitt (21b) 4 bis 8, insbesondere 8, zweite Stoffaustauschböden (100) aufweist.

10. Verfahren nach einem der vorhergehenden Ansprüche, **dadurch gekennzeichnet, dass** die Ölwaschkolonne (1) einen im Ölabschnitt (21) angeordneten ersten Flüssigkeitsverteiler (200) aufweist, mit dem die zweiten Stoffaustauschböden (100) mit dem zweiten Waschmittel (W') beaufschlagt werden, wobei der erste Flüssigkeitsverteiler (200) jenes zweite Waschmittel (W') auf die Kanten (104) der Ablaufelemente (101) eines obersten zweiten Stoffaustauschbodens (200) des Ölabschnitts (21) aufgibt, so dass jenes zweite Waschmittel (W') zu beiden Seiten der jeweiligen Kante (104) über die Ablaufflächen (102a, 103a) vom jeweiligen Ablaufelement (101) abfließt, wobei insbesondere der erste Flüssigkeitsverteiler (200) im Leichtölabschnitt (21a) angeordnet ist und jener oberste zweite Stoffaustauschboden (100) ein oberster zweiter Stoffaustauschboden (100) des Leichtölabschnitts (21a) ist, und wobei insbesondere ein zweiter Flüssigkeitsverteiler (300) im Schwerölabschnitt (21b) angeordnet ist, mit dem die zweiten Stoffaustauschböden (100) des Schwerölabschnitts (21b) mit jenem dritten Waschmittel (W") beaufschlagt werden, und wobei insbesondere der zweite Flüssigkeitsverteiler (300) jenes dritte Waschmittel (W") auf die Kanten (104) der Ablaufelemente (101) eines obersten zweiten Stoffaustauschbodens (100) des Schwerölabschnitts (21b) aufgibt, so dass jenes dritte Waschmittel (W") zu beiden Seiten der jeweiligen Kante (104) über die Ablaufflächen (102a, 103a) vom jeweiligen Ablaufelement (101) abfließt.

11. Verfahren nach Anspruch 10, **dadurch gekennzeichnet, dass** der erste und/oder der zweite Flüssigkeitsverteiler (200, 300) jeweils eine Mehrzahl an Auslässen (201) aufweisen, durch die hindurch das jeweilige Waschmittel (W', W") auf die Kanten (104) der Ablaufelemente (101) des jeweiligen obersten zweiten Stoffaustauschbodens (100) aufgegeben wird, wobei die Auslässe (201) jeweils lotrecht oberhalb einer Kante (104) eines zugeordneten Ablaufelementes (101) angeordnet sind.

12. Verfahren nach Anspruch 10 oder 11, **dadurch gekennzeichnet, dass** der erste und/oder der zweite Flüssigkeitsverteiler (200, 300) jeweils eine Mehrzahl an Endverteilerkanälen (202) aufweisen, die sich jeweils entlang des besagten Kolonnenquerschnitts (Q) sowie quer zu den Ablaufelementen (101) erstrecken.

13. Verfahren nach Anspruch 12, **dadurch gekennzeichnet, dass** die Endverteilerkanäle (202) jeweils einen quer zur Längsachse (L) erstreckten Boden (203) und zwei davon abgehende Seitenwände (204) aufweisen, wobei die besagten Seitenwände (204) jeweils einen oberen Rand (205) aufweisen, und wobei die Auslässe (201) in Form von insbesondere rechteckförmigen Ausnehmungen an den beiden oberen Rändern (205) ausgebildet sind.

14. Verfahren nach Anspruch 13, **dadurch gekennzeichnet, dass** der erste und/oder der zweite Flüssigkeitsverteiler (200, 300) zumindest zwei zueinander parallele, entlang der Längsachse (L) oberhalb der Endverteilerkanäle (202) angeordnete Vorverteilerkanäle (210) aufweisen, mit denen die Endverteilerkanäle (202) mit dem jeweiligen Waschmittel (W', W") beschickt werden, wobei sich die Vorverteilerkanäle (210) insbesondere entlang des Kolonnenquerschnitts (Q) erstrecken, und wobei insbesondere die Vorverteilerkanäle (210) quer zu den Endverteilerkanälen (202) verlaufen.

15. Verfahren nach Anspruch 14, **dadurch gekennzeichnet, dass** die Vorverteilerkanäle (210) jeweils einen quer zur Längsachse (L) erstreckten Boden (211) und zwei davon abgehende Seitenwände (212) aufweisen, wobei die besagten Seitenwände (212) jeweils einen oberen Rand (213) aufweisen, an denen Auslässe (214) in Form von insbesondere rechteckförmigen Ausnehmungen ausgebildet sind, über die das jeweilige Waschmittel (W', W") in jeweils einen zugeordneten Endverteilerkanal (202) eingeleitet wird, wobei insbesondere jene Auslässe (214) der Vorverteilerkanäle (210) jeweils lotrecht oberhalb eines zugeordneten Endverteilerkanals (202) angeordnet sind, wobei insbesondere die Vorverteilerkanäle (210) über zumindest einen Ausgleichskanal (215) miteinander verbunden sind.

## Claims

1. Process for the purification of a cracking gas stream in an oil scrub column which has a shell (10) which is extended along a longitudinal axis (L) and encloses an interior (I) of the column (1), the interior (I) being subdivided into a petroleum spirit section (20) and into an oil section (21), which is arranged below the petroleum spirit section (20) along the longitudinal axis (L), where a cracking gas stream (S) is passed into the oil section (21) and guided from bottom to top along the longitudinal axis (L) through second mass transfer trays (100) of the oil section (21) which are arranged one above another and which are subjected to a liquid, hydrocarbon-containing second scrubbing medium (W') in countercurrent with respect to the cracking gas stream (S), in order to separate out an oil fraction (F') from the cracking gas stream (S), where the second mass transfer trays (100) each have a plurality of runoff elements (101) extending parallel to and at a distance from one another, more particularly in the form of angular profiles, which each have a first and a second runoff surface (102a, 103a), along which the second scrubbing medium (W') runs downward, and which converge along the longitudinal axis (L) towards the petroleum spirit section and meet, and in so doing form an edge (104) extending transversely to the longitudinal axis (L), where the cracking gas stream, after traversing the second mass transfer trays (100) of the oil section (21), is passed into the petroleum spirit section (20), and is then guided along the longitudinal axis (L) from bottom to top through first mass transfer trays (16a, 16b, 16c) of the petroleum spirit section (20), these trays being arranged one above another and taking the form of sieve trays, bubble trays and/or valve trays, and being fed, in countercurrent to the cracking gas stream (S), with a liquid, hydrocarbon-containing first scrubbing medium (W), in order to remove a petroleum spirit fraction (F) from the cracking gas stream (S), where the cracking gas stream (S) is drawn off from a top (11) of the petroleum spirit section (20), where this petroleum spirit fraction (F) is drawn off from the petroleum spirit section (20), and where a petroleum spirit fraction (W) is recycled as first scrubbing medium (W) into the top (11) of the petroleum spirit section (20),
**characterized**
**in that** the molar ratio of the amount of substance of the petroleum spirit fraction (W) recycled into the petroleum spirit section (20) per unit time to the amount of substance of the cracking gas (S) introduced into the oil section (21) per unit time is in a range from 1:16 to 1:10, preferably 1:12 to 1:10.

2. Process according to Claim 1, **characterized in that** a portion of the petroleum spirit fraction (F) drawn off from the petroleum spirit section (20) is recycled onto one of the first mass transfer trays (16b, 16c), more particularly onto one of the lower first mass transfer trays (16b, 16c) .

3. Process according to Claim 2, **characterized in that** this first mass transfer tray is the second-lowermost (16b), third-lowermost (16c) or fourth-lowermost first mass transfer tray.

4. Process according to any of the preceding claims, **characterized in that** the petroleum spirit fraction (F) is drawn off from a chimney tray (13) or from a lowermost first mass transfer tray that separates the petroleum spirit section (20) from the oil section (21).

5. Process according to any of the preceding claims, **characterized in that** the oil section (21) is subdivided into a light oil section (21a) and into a heavy oil section (21b), which is arranged below section (21a) along the longitudinal axis (L), there being arranged in each of these sections a plurality of second mass transfer trays (100), where in particular the light oil section (21a) is separated from the heavy oil section (21b) by means of a chimney tray (14), and where the cracking gas stream (S) is guided through the second mass transfer trays (100) of the heavy oil section (21b) and subsequently through the second mass transfer trays (100) of the light oil section (21a), where the second mass transfer trays (100) in the heavy oil section (21b) are subjected to a liquid, hydrocarbon-containing third scrubbing medium (W"), in order to separate out a heavy oil fraction (F'') from the cracking gas stream (S), and where the second transfer trays (100) in the light oil section (21a) are subjected to a liquid, hydrocarbon-containing second scrubbing medium (W'), in order to separate out a light oil fraction (F') from the cracking gas stream (S).

6. Process according to any of the preceding claims, **characterized in that** the petroleum spirit section (20) has 6 to 8, more particularly 7, first mass transfer trays (16a, 16b, 16c), where, in particular, adjacent first mass transfer trays have a distance from one another along the longitudinal axis (L) in the range from 500 mm to 900 mm.

7. Process according to any of the preceding claims, **characterized in that** the oil section (21) has 10 to 20, more particularly 16, second mass transfer trays (100).

8. Process according to Claim 6, **characterized in that** the light oil section (21a) has 6 to 12, more particularly 8, second mass transfer trays (100).

9. Process according to Claim 6 or 8, **characterized in that** the heavy oil section (21b) has 4 to 8, more particularly 8, second mass transfer trays (100).

10. Process according to any of the preceding claims, **characterized in that** the oil scrub column (1) has a first liquid distributor (200), which is arranged in the oil section (21) and by means of which the second mass transfer trays (100) are subjected to the second scrubbing medium (W'), where the first liquid distributor (200) introduces this second scrubbing medium (W') onto the edges (104) of the runoff elements (101) of an uppermost second mass transfer tray (200) of the oil section (21), so that this second scrubbing medium (W') flows off from the respective runoff element (101) via the runoff surfaces (102a, 103a) at both sides of the respective edge (104), where in particular the first liquid distributor (200) is arranged in the light oil section (21a) and this uppermost second mass transfer tray (100) is an uppermost second mass transfer tray (100) of the light oil section (21a), and where in particular a second liquid distributor (300) is arranged in the heavy oil section (21b), by means of which second liquid distributor (300) the second mass transfer trays (100) of the heavy oil section (21b) are subjected to this third scrubbing medium (W"), and where in particular the second liquid distributor (300) introduces this third scrubbing medium (W") onto the edges (104) of the runoff elements (101) of an uppermost second mass transfer tray (100) of the heavy oil section (21b), so that this third scrubbing medium (W") flows off from the respective runoff element (101) via the runoff surfaces (102a, 103a) at both sides of the respective edge (104).

11. Process according to Claim 10, **characterized in that** the first and/or the second liquid distributors (200, 300) each have a plurality of cutouts (201) through which the respective scrubbing medium (W', W") is introduced onto the edges (104) of the runoff elements (101) of the respective uppermost second mass transfer tray (100), where the cutouts (201) are each arranged perpendicularly above an edge (104) of an assigned runoff element (101).

12. Process according to Claim 10 or 11, **characterized in that** the first and/or the second liquid distributors (200, 300) each have a plurality of final distributor channels (202) which are each extended along said column cross section (Q) and also transversely to the runoff elements (101).

13. Process according to Claim 12, **characterized in that** the final distributor channels (202) each have a base (203) which is extended transversely to the longitudinal axis (L), and two side walls (204) starting from said base (203), where said side walls (204) each have an upper rim (205), and where the cutouts (201) are designed in the form of vacancies, more particularly rectangular vacancies, at the two upper rims (205).

14. Process according to Claim 13, **characterized in that** the first and/or second liquid distributors (200, 300) have at least two preliminary distributor channels (210), which are parallel to one another, are arranged above the final distributor channels (202) along the longitudinal axis (L) and by means of which the final distributor channels (202) are charged with the respective scrubbing medium (W', W"), where the preliminary distributor channels (210) are extended more particularly along the column cross section (Q), and where in particular the preliminary distributor channels (210) extend transversely to the final distributor channels (202).

15. Process according to Claim 14, **characterized in that** the preliminary distributor channels (210) each have a base (211) which is extended transversely to the longitudinal axis (L), and two side walls (212) starting from said base (211), where said side walls (212) each have an upper rim (213), at which cutouts (214) are designed in the form of vacancies, more particularly rectangular vacancies, via which the respective scrubbing medium (W', W") is led into respectively one assigned final distributor channel (202), where in particular these cutouts (214) of the preliminary distributor channels (210) are arranged in each case perpendicularly above an assigned final distributor channel (202), where in particular the preliminary distributor channels (210) are connected to one another via at least one compensation channel (215).

## Revendications

1. Procédé de purification d'un courant de gaz de craquage dans une colonne de lavage d'huile, qui comprend une enveloppe (10) le long d'un axe longitudinal (L), qui entoure un espace intérieur (I) de la colonne (1), l'espace intérieur (I) étant divisé en une section d'essence (20) et une section d'huile (21) agencée en dessous le long de l'axe longitudinal (L), un courant de gaz de craquage (S) étant introduit dans la section d'huile (21) et acheminé le long de l'axe longitudinal (L) du bas vers le haut au travers de deuxièmes plateaux d'échange de matière superposés (100) de la section d'huile (21), qui sont chargés à contre-courant du courant de gaz de craquage (S) avec un deuxième agent de lavage liquide contenant des hydrocarbures (W'), afin de séparer une fraction d'huile (F') du courant de gaz de craquage (S), les deuxièmes plateaux d'échange de matière (100) comprenant chacun une pluralité d'éléments d'écoulement (101) parallèles et espacés les uns des autres, notamment sous la forme de cornières, qui comprennent chacun une première et une deuxième surface d'écoulement (102a, 103a), le long desquelles le deuxième agent de lavage (W') s'écoule vers le bas et qui s'approchent l'une de l'autre le long de l'axe longitudinal (L) vers la section d'essence et convergent en formant un bord (104) perpendiculaire à l'axe longitudinal (L), le courant de gaz de craquage étant introduit dans la section d'essence (20) après avoir traversé les deuxièmes plateaux d'échange de matière (100) de la section d'huile (21), puis étant acheminé le long de l'axe longitudinal (L) du bas vers le haut au travers de premiers plateaux d'échange de matière superposés (16a, 16b, 16c) de la section d'essence (20), qui sont configurés sous la forme de plateaux perforés, de plateaux à calottes et/ou de plateaux à soupapes, et qui sont chargés à contre-courant du courant de gaz de craquage (S) avec un premier agent de lavage liquide contenant des hydrocarbures (W), afin de séparer une fraction d'essence (F) du courant de gaz de craquage (S), le courant de gaz de craquage (S) étant soutiré à partir d'une tête (11) de la section d'essence (20), cette fraction d'essence (F) étant soutirée de la section d'essence (20) et une fraction d'essence (W) étant recyclée en tant que premier agent de lavage (W) dans la tête (11) de la section d'essence (20),
**caractérisé en ce que**
le rapport molaire entre la quantité de matière de la fraction d'essence (W) recyclée dans la section d'essence (20) par unité de temps et la quantité de matière du gaz de craquage (S) introduite par unité de temps dans la section d'huile (21) se situe dans une plage allant de 1:16 à 1:10, de préférence de 1:12 à 1:10.

2. Procédé selon la revendication 1, **caractérisé en ce qu'**une partie de la fraction d'essence (F) soutirée de la section d'essence (20) est recyclée sur un des premiers plateaux d'échange de matière (16b, 16c), notamment sur un des premiers plateaux d'échange de matière inférieurs (16b, 16c).

3. Procédé selon la revendication 2, **caractérisé en ce que** le premier plateau d'échange de matière est le deuxième (16b), le troisième (16c) ou le quatrième premier plateau d'échange de matière le plus inférieur.

4. Procédé selon l'une quelconque des revendications précédentes, **caractérisé en ce que** la fraction d'essence (F) est soutirée à partir d'un plateau à cheminées (13) ou d'un premier plateau d'échange de matière le plus inférieur, qui sépare la section d'essence (20) de la section d'huile (21).

5. Procédé selon l'une quelconque des revendications précédentes, **caractérisé en ce que** la section d'huile (21) est divisée en une section d'huile légère (21a) et une section d'huile lourde (21b) agencée en dessous le long de l'axe longitudinal (L), dans lesquelles plusieurs deuxièmes plateaux d'échange de matière (100) sont agencés à chaque fois, la section d'huile légère (21a) étant notamment séparée de la section d'huile lourde (21b) au moyen d'un plateau à cheminées (14), et le courant de gaz de craquage (S) étant acheminé au travers des deuxièmes plateaux d'échange de matière (100) de la section d'huile lourde (21b), puis au travers des deuxièmes plateaux d'échange de matière (100) de la section d'huile légère (21a), les deuxièmes plateaux d'échange (100) dans la section d'huile lourde (21b) étant chargés avec un troisième agent de lavage liquide contenant des hydrocarbures (W"), afin de séparer une fraction d'huile lourde (F") du courant de gaz de craquage (S), et les deuxièmes plateaux d'échange (100) dans la section d'huile légère (21a) étant chargés avec un deuxième agent de lavage liquide contenant des hydrocarbures (W'), afin de séparer une fraction d'huile légère (F') du courant de gaz de craquage (S).

6. Procédé selon l'une quelconque des revendications précédentes, **caractérisé en ce que** la section d'essence (20) comprend 6 à 8, notamment 7, premiers plateaux d'échange de matière (16a, 16b, 16c), des premiers plateaux d'échange de matière voisins présentant notamment un écart entre eux le long de l'axe longitudinal (L) dans la plage allant de 500 mm à 900 mm.

7. Procédé selon l'une quelconque des revendications précédentes, **caractérisé en ce que** la section d'huile (21) comprend 10 à 20, notamment 16, deuxièmes plateaux d'échange de matière (100).

8. Procédé selon la revendication 6, **caractérisé en ce que** la section d'huile légère (21a) comprend 6 à 12, notamment 8, deuxièmes plateaux d'échange de matière (100).

9. Procédé selon la revendication 6 ou 8, **caractérisé en ce que** la section d'huile lourde (21b) comprend 4 à 8, notamment 8, deuxièmes plateaux d'échange de matière (100).

10. Procédé selon l'une quelconque des revendications précédentes, **caractérisé en ce que** la colonne de lavage d'huile (1) comprend un premier distributeur de liquide (200) agencé dans la section d'huile (21), avec lequel les deuxièmes plateaux d'échange de matière (100) sont chargés avec le deuxième agent de lavage (W'), le premier distributeur de liquide (200) distribuant le deuxième agent de lavage (W') sur les bords (104) des éléments d'écoulement (101) d'un deuxième plateau d'échange de matière le plus supérieur (200) de la section d'huile (21), de sorte que le deuxième agent de lavage (W') s'écoule des deux côtés du bord respectif (104) sur les surfaces d'écoulement (102a, 103a) de l'élément d'écoulement respectif (101), le premier distributeur de liquide (200) étant notamment agencé dans la section d'huile légère (21a) et le deuxième plateau d'échange de matière le plus supérieur (100) étant un deuxième plateau d'échange de matière le plus supérieur (100) de la section d'huile légère (21a), et un deuxième distributeur de liquide (300) étant notamment agencé dans la section d'huile lourde (21b), avec lequel les deuxièmes plateaux d'échange de matière (100) de la section d'huile lourde (21b) sont chargés avec le troisième agent de lavage (W"), le deuxième distributeur de liquide (300) distribuant le troisième agent de lavage (W") sur les bords (104) des éléments d'écoulement (101) d'un deuxième plateau d'échange de matière le plus supérieur (100) de la section d'huile lourde (21b), de sorte que le troisième agent de lavage (W") s'écoule des deux côtés du bord respectif (104) sur les surfaces d'écoulement (102a, 103a) de l'élément d'écoulement respectif (101).

11. Procédé selon la revendication 10, **caractérisé en ce que** le premier et/ou le deuxième distributeur de liquide (200, 300) comprennent chacun une pluralité de sorties (201), par lesquelles l'agent de lavage respectif (W', W") est distribué sur les bords (104) des éléments d'écoulement (101) du deuxième plateau d'échange de matière le plus supérieur respectif (100), les sorties (201) étant chacune agencées à la verticale au-dessus d'un bord (104) d'un élément d'écoulement correspondant (101) .

12. Procédé selon la revendication 10 ou 11, **caractérisé en ce que** le premier et/ou le deuxième distributeur de liquide (200, 300) comprennent chacun une pluralité de canaux de distribution finaux (202), qui s'étendent chacun le long de ladite section transversale de la colonne (Q) et perpendiculairement aux éléments d'écoulement (101).

13. Procédé selon la revendication 12, **caractérisé en ce que** les canaux de distribution finaux (202) comprennent chacun un fond (203) perpendiculaire à l'axe longitudinal (L) et deux parois latérales (204) partant de celui-ci, lesdites parois latérales (204) comprenant chacune un rebord supérieur (205) et les sorties (201) étant configurées sous la forme d'ouvertures notamment rectangulaires sur les deux rebords supérieurs (205).

14. Procédé selon la revendication 13, **caractérisé en ce que** le premier et/ou le deuxième distributeur de liquide (200, 300) comprennent au moins deux canaux de pré-alimentation (210) parallèles les uns aux autres, agencés le long de l'axe longitudinal (L) au-dessus des canaux de distribution finaux (202), avec lesquels les canaux de distribution finaux (202) sont alimentés avec l'agent de lavage respectif (W', W"), les canaux de pré-alimentation (210) s'étendant notamment le long de la section transversale de la colonne (Q) et les canaux de pré-alimentation (210) étant notamment perpendiculaires aux canaux de distribution finaux (202).

15. Procédé selon la revendication 14, **caractérisé en ce que** les canaux de pré-alimentation (210) comprennent chacun un fond (211) perpendiculaire à l'axe longitudinal (L) et deux parois latérales (212) partant de celui-ci, lesdites parois latérales (212) comprenant chacune un rebord supérieur (213), sur lequel des sorties (214) sont configurées sous la forme d'ouvertures notamment rectangulaires, par lesquelles l'agent de lavage respectif (W', W") est introduit à chaque fois dans un canal de distribution final correspondant (202), les sorties (214) des canaux de pré-alimentation (210) étant notamment agencées chacune à la verticale au-dessus d'un canal de distribution final correspondant (202), les canaux de pré-alimentation (210) étant notamment reliés les uns avec les autres par au moins un canal d'égalisation (215).
